# EUROPEAN PATENT APPLICATION

(11) **EP 4 696 782 A1**
(43) Date of publication of application: **18.02.2026**
(21) Application number: 24194548.4
(22) Date of filing: 14.08.2024
(51) Int. Cl.: C12N 15/86, A61K 39/12

(54) **IMPROVED SPLIT RNA REPLICONS**

(71) Applicant: Starvax One B.V., 5865 BR Tienray (NL)
(72) Inventor: STADLER, Konrad, 5865 BR Tienray (NL); SEIDLER, Randolph Wilfried Richard, 5865 BR Tienray (NL); SNO-GUBBELS, Melanie Maria Roberta, 5865 BR Tienray (NL); LEMON, Kenneth, BT9 5PX Belfast (GB)
(74) Representative: Bianchetti & Minoja with Trevisan & Cuonzo IPS SRL

(57) **Abstract**

The invention embraces an advantageous split replicon system allowing easy, plug-and-play, multivalency of RNA-therapeutics as well as RNA-vaccines. The system as provided herein comprises combining essentially purified RNA derived from a positive strand RNA virus and encoding self-amplifying or self-replicating RdRp in a split-replicon fashion with one or more essentially purified RNAs having a GOI, or different GOIs inserted in the area of the RdRp-gene(s), and preferably structurally deleting viral structural proteins by avoiding use of nucleic acid encoding said structural protein(s) all together, and therewith rendering the system small, versatile, safe and propagation defective.

## Description

### Field of the invention

The invention relates to the field of RNA-replicon therapeutics. Vaccines are recognized to be one of the most cost-effective interventions for the prevention of infectious diseases. Effective and safe vaccines capable of undergoing mass production provide the prospect of eradication of certain infectious diseases. In most cases, a large proportion of vaccination scheme costs arises from maintaining cold chains, storage, and transport as well as the salaries of medical and paramedical staff rather than the costs of the vaccines themselves. Despite this, some recently introduced advanced vaccines are markedly more costly. The expenditure on vaccination programs can be minimized by the well-established practice of combining individual vaccines (e.g., diphtheria/tetanus/pertussis or mumps/measles/rubella), but each component must be manufactured separately, and the method of combination can be complex. With the advent of genetically engineered viral vaccines, it has become feasible to combine multiple protective antigens into multivalent vaccines that are useful tools to protect from cocirculating pathogens as a greater number of protective antigens are presented to the immune system. The seasonal influenza vaccine which is constituted from three and four different HA antigens, respectively, corresponding to the circulating influenza virus A and B strains is an example a a commonly used multivalent vaccine. Multivalent mRNA vaccines are currently in development but tend to run into safety issues which the current invention avoid.

Recent advances in neoantigen research have accelerated the development and regulatory approval of tumour immunotherapies, including cancer vaccines. Neoantigens are newly formed antigens generated by tumour cells because of various tumour-specific alterations. The accumulation of genetic alterations in cancers results in the production of tumour-specific antigens (TSAs) or neoantigens, which can be presented by major histocompatibility (MHC) molecules of tumour cells. These tumour-specific peptide-MHC (pMHC) complexes are recognized by T cells and trigger an anti-cancer immune response in patients. However, it has been discovered that cancer cells have evolved resistance to anti-cancer immunity. These immune escape mechanisms can be reversed by tumour vaccines to improve antigen presentation. The highly immunogenic and tumour-specific neoantigens provide emerging targets for personalized cancer vaccines.

### Background

As reviewed recently (Gomes, Pijlman and Hick, Rise of the RNA machines - self-amplification in mRNA vaccine design Trends Biotechnol 2023 Jun 14), mRNA vaccines have won the race for early COVID-19 vaccine approval, yet improvements are necessary to retain this leading role in combating infectious diseases. A next generation of self-amplifying mRNAs, also known as replicons, are found to form an ideal vaccine platform. The genomes of positive sense RNA viruses are infectious in the absence of any viral proteins. The infectious "heart" of a positive-strand RNA virus is its genome. Therefore, the genomes of many positive-strand RNA virus can be synthesized and introduced into a cell with the aim of producing replicating units such as replicons. Replicon RNA resembles *in vitro* transcribed mRNA, but additionally encodes viral replicase genes. These genes allow the rapid amplification of the mRNA and thereby increase the production of the GOI in comparison to non-amplifying mRNAs. The massive rollout of mRNA vaccines revealed challenges in balancing the high administration dose with adverse effects, the requirement of prime-boost vaccinations, and the necessity of cold-chain storage. These challenges can be overcome by the next generation of mRNA vaccines based on self-amplifying mRNA, also known as replicon RNA. Both conventional mRNA and replicating RNA vaccine approaches typically share essential elements of eukaryotic mRNA: a cap structure (cap 0, 1, or 2), a 5' UTR, an open reading frame (ORF), a 3' UTR, and a tail of 40-120+ adenosine residues [poly(A) tail]. Both types of RNAs are typically produced in a cell-free system of in vitro transcription, if required using modified nucleosides, by using an enzymatic transcription reaction from a linearized DNA template requiring a DNA-dependent RNA polymerase. Manufacturing of RNA vaccines against different disease targets requires the replacement of the sequence encoding the target antigen encoded in the ORF, possibly affecting the overall physicochemical and replicative characteristics of the RNA molecule. Several RNA replicon vaccines of this kind have been reported

Whereas mRNA vaccines typically solely encode a gene-of-interest (GOI) and several (flanking) recognition sequences (RS) required for expression of the desired protein-of-interest, self-amplifying RNA (saRNA) molecules have been engineered as a single molecular chassis encoding the gene of interest (GOI) as well as all essential *cis*-acting elements allowing self-amplification of the replicon RNA within a cell. The rapid amplification of replicon RNA in target cells increases the expression of the protein of interest (e.g., a viral (glyco)protein) and induces a protective immune response at a markedly lower initial RNA dose than conventional mRNA vaccines. Historically, positive-sense single-stranded RNA viruses, such as alphaviruses, flaviviruses, and picornaviruses have been used for saRNA replicons (Blakney, A.K.; Ip, S.; Geall, A.J. An Update on Self-Amplifying mRNA Vaccine Development. Vaccines 2021, 9, 97.).

The genome of a positive strand RNA virus, unlike that of any other virus or organism, must function both as the repository of genetic information (i.e., as the genome) and as a messenger RNA. Thus, the virus must coordinate the mutually exclusive activities of translation, RNA replication and encapsidation, all of which occur on the same genomic RNA molecule, but not at the same time. In addition, for many positive strand RNA viruses, the conflicting activities of full-length genome synthesis and subgenomic mRNA transcription from the same minus strand template must be balanced. All of these functions must be stringently regulated to ensure that the appropriate amounts of each viral protein and each viral RNA are produced at the right times and in the right subcellular locations to facilitate a productive infection cycle. Almost the entire genome of a positive strand RNA is occupied by (sometimes overlapping) open reading frames encoding the viral proteins which leaves the main burden of regulating all of the complicated and interacting processes of the virus replication cycle mainly up to the untranslated regions at the 5' and 3' ends of the viral genome. However, terminal regulatory sequences and structures sometimes extend into the coding regions, and in a few cases, are also located in internal (subgenomic) regions of the genome. In summary, the importance of the ends of the genomes as master controllers of the positive strand RNA virus life cycle cannot be over emphasized and typically replicon systems follow the adage that a gene-of-interest is to be inserted in genomic areas of the viral RNA where structural proteins are encoded, therewith taking care of at least functionally deleting structural proteins to render the resulting replicon propagation defective as well as at the same time providing a convenient location for insertion a said gene-of-interest.

Many (+)-strand RNA viruses use subgenomic (SG) RNAs as messengers for protein expression, or to regulate their viral life cycle. Three different mechanisms have been described for the synthesis of SG RNAs. The first mechanism involves internal initiation on a (-)-strand RNA template and requires an internal SGP promoter. The second mechanism makes a prematurely terminated (-)-strand RNA which is used as template to make the SG RNA. The third mechanism uses discontinuous RNA synthesis while making the (-)-strand RNA templates. Most SG RNAs are translated into structural proteins or proteins related to pathogenesis: however other SG RNAs regulate the transition between translation and replication, function as riboregulators of replication or translation, or support RNA-RNA recombination. Following the synthesis of the viral RNA-dependent RNA polymerase (RdRp), the (+) strand RNA is copied into a genome-length (-) strand which then serves as a template for the genomic (G) and the SG (+) strand RNAs. Thus, then the (-) strand RNA contains at least two different promoters, one for the synthesis of genomic RNA at or near the 3' end, and one or more internal or subgenomic promoters (SGPs). To synthesize a subgenomic (SG) RNA, the viral RdRp recognizes and binds to the SGP and initiates transcription.

The best-studied self-amplifying mRNA molecules are derived from alphavirus genomes that have a bicistronic genome wherein the open reading frame encoding the non-structural proteins is separated by an untranslated region from the open reading frame encoding the structural proteins, and alpha- and flavivirus-based replicons are best studied for both human and veterinary applications. Alphavirus-based replicons typically contain a separated open-reading frame (ORF), that encodes the non-structural replicase proteins, upstream of the (at least partially deleted) area of alpha-virus structural protein-location where the GOI of the saRNA-replicon is located, and typically downstream of the (endogenous) subgenomic promotor (SGP) that facilitates structural protein transcription and translation. In contrast, in flavivirus-based replicons the non-structural replicase proteins are encoded in a single ORF downstream of the (at least partially deleted) area of structural protein-location where the GOI of the saRNA replicon is located. Since replicon saRNA does not encode all alphavirus or flavivirus structural proteins, the RNA is propagation defective.

As a result, replicon (saRNA) vaccines are, like the non-amplifying mRNA vaccines, categorized as synthetic nucleic acid vaccines. Because viral structural genes have been replaced by a gene-of-interest, the replicon RNA cannot spread in the environment, which is a key difference with chimeric or recombinant virus vaccines. Typically, both upstream as well as downstream from the GOI-area, various conserved sequence elements (CSE's) are maintained in the replicon that serve to facilitate viral replicase activities that in the virus of origin are found to flank (and sometimes partly overlap) the nucleotides encoding the structural proteins of the respective viruses.

A further development in the saRNA field comprise combining replicase-coding mRNA that in itself is non-replicating with an RNA-replicon having a GOI inserted in the area of the structural protein genes. A first example is the splitzicon system [Blakney, A.K.; McKay, P.F.; Shattock, R.J. Structural Components for Amplification of Positive and Negative Strand VEEV Splitzicons. Front. Mol. Biosci. 2018, 5, 71] wherein the replicase proteins are provided from a non-amplifying RNA (RdRp-gene) encoding the non-structural proteins (NSP) without providing a 3'UTR and/or a 3' polyA-tail to said gene that would provide for negative strand formation. The studied reporter luciferase gene of interest is encoded on separate RNA molecules, 11 in a positive strand fashion, and 3 in a negative strand fashion. Only one of the 11 positive strand combinations (Pos 6 with the reporter gene inserted downstream from the subgenomic reporter (SGP) in the structural protein area), and all 3 negative strands (each derived from said Pos 6) exhibit a dose-depended-amplification by the NSP provided. Fully in line with this splitzicon work, Beissert et al (Mol Ther. 2020 Jan 8;28(1): 119-128, see also WO2017162461) discuss a split replicon RNAvaccine approach based on an alphaviral-derived bipartite vector system using trans-amplifying RNA (taRNA). The vector cassette encoding the vaccine antigen originates from an alphaviral replicating RNA, from which the replicase was deleted to form a transreplicon and the GOI was inserted in the area downstream from the subgenomic promotor (SGP) . The alphavirus replicase activity is provided in trans by a second molecule, either by a standard replicating RNA having a so-called irrelevant transgene (iTG) inserted downstream of the subgenomic promotor or an optimized non-replicating mRNA (nrRNA). Typically, Beissert et al found the non-replicating RNA delivered 10- to 100-fold higher transreplicon (TR) expression than the standard replicating RNA carrying the iTG in the area of the structural proteins. Moreover, expression driven by the nrRNA-encoded replicase in this split replicon RNA system was nearly as efficient as in a conventional monopartite RNA system whereas the bipartite RNA system using the replicating RNA to encode the replicase performed considerably less well. Beissert et al (ibid) show that a superiority of nrRNA- over replicating RNA-encoded replicase to drive expression of the transreplicon is most likely attributable to its higher translational efficiency and lack of interference with cellular translation. Beissert et al are also introducing the GOI of the transreplicon in the position of the structural proteins downstream from said SGP. Expression driven by the nrRNA-encoded replicase in the taRNA system was found as efficient as a conventional monopartite saRNA system in a mouse influenza challenge model.

The family *Nodaviridae* (herein also nodavirus) consists of at least three genera, the Alphanodavirus genus including Nodamuravirus (NoV), Flock House virus (FHV), and Black beetle virus (BBV) that primarily infect insect cells, the Betanodaviruses that mainly infect fish, and the Noda-like viruses. An interesting aspect of the nodaviruses is that they can replicate in several different cell types outside of those they tend to naturally infect. Nodaviruses have a bipartite genome wherein the open reading frame encoding the non-structural proteins is located on a separate RNA-strand from the open reading frame encoding the structural proteins. Nodaviruses contain this bipartite single-stranded positive-sense RNA genome [Ball LA, Johnson KL. 1999. Reverse genetics of nodaviruses. Adv. Virus Res. 53:229-244], essentially comprising two different RNA-molecules, aptly identified as RNA1 and RNA2 segments. RNA1 encodes the 110 kD RNA-dependent RNA polymerase (RdRp), which replicates the genomic RNA1 and RNA2 and the subgenomic RNA3 segments located in RNA1 via negative strand RNA intermediates. RNA2 encodes the viral capsid protein (CP) and is dispensable for RNA1 replication. In the viral life-cycle, the RdRp binds to and replicates each of these two RNA-molecules, and the CP packages the two of them into a single capsid [Ball LA, Amann JM, Garrett BK. 1992.. Replication of Nodamura virus after transfection of viral RNA into mammalian cells in culture. J. Virol. 66:2326-2334]. During RNA1 replication a subgenomic RNA3 from RNA1 is synthesized overlapping with the 3' end of RNA1. RNA3 encodes 2 viral proteins, B1 and B2. B2, and possibly also B1,suppresses RNA interference RNAi [Sullivan CS, Ganem D. 2005. A virus-encoded inhibitor that blocks RNA interference in mammalian cells. J. Virol. 79:7371-7379]. In addition, RNA3 reportedly has been shown to be required for RNA2 replication [Roskopf, thesis 2009. Cis-acting signals for replication of Nodamura virus RNA1]. RNA secondary structures present in viral genomes play a role in a variety of processes in the viral life cycle. These structures are often found in the untranslated regions (UTR's) of viral RNA and play roles in viral RNA replication, translation, and protection from nucleolytic digestion.

Joyner [thesis 2022. A study of *in cis* versus *in trans* viral RNA replication: RNA molecules competing to be replicated by an RNA replicase protein] assessed the relative levels at which the two different RNA1 and RNA2 molecules compete to be bound and replicated by the RdRp. In vitro transcribed Nodamura RNA constructs were created in which a first fluorescent reporter gene is added at the 3' end of RNA1 partially replacing information for RNA3, whereas the CP gene in RNA2 was replaced entirely by a different, second, fluorescent reporter gene. These two constructs were then transfected into mammalian (BHK-21) cells. Fluorescence intensity assays and quantitative PCR experiments were performed at various time points post-transfection to study the replication competition between Nodamura's RNA1 and RNA2 in the absence and presence of one another, each respectively having their own specific 3' UTRs derived from RNA1 and RNA2. These assays showed that Nodamura RNA1 replication decreases by as much as a factor of two when in the presence of RNA2, while RNA2 was found not amplified at all in the presence of the RdRp encoded by RNA1. While significant levels of *in cis* replication were anticipated to be observed in these experiments, the near complete lack of *in trans* replication of RNA2 was quite surprising. Joyner's results indicate how *in cis* and *in trans* replication dynamics may determine the differential expression of the viral genomic RNAs in quite unpredictable fashion.

FHV, BBV and NoV are functionally related, e.g. the RdRp from both viruses fulfill the same functions and are both transmembrane located proteins [Miller DJ, Ahlquist P. 2002. Flock House virus RNA polymerase is a transmembrane protein with amino-terminal sequences sufficient for mitochondrial localization and membrane insertion. J. Virol. 76:9856-9867.], however, FHV and NoV share only 44% sequence identity on the amino acid level [Price BD, Ahlquist P, Ball LA. 2002. DNA-directed expression of an animal virus RNA for replication-dependent colony formation in Saccharomyces cerevisiae. J. Virol. **76**:1610-1616]. Also, their enzymatic activity differs and NoV RdRp is more thermostabel and active at 37°C while FHV RdRp becomes inactive at temperatures above 31°C [Ball LA, Amann JM, Garrett BK. 1992. Replication of Nodamura virus after transfection of viral RNA into mammalian cells in culture. J. Virol. 66:2326-2334]. As with all wild-type RNA viruses, nodaviruses also often form complex distributions of closely related but nonidentical variant genomes that are subjected to a continuous process of genetic variation, competition, and selection [Martinez et al., Quasispecies Dynamics of RNA Viruses. Viruses: Essential Agents of Life. 2012;21-42]. These so-called viral quasispecies have been described in vivo through the analysis of molecular and biological clones isolated from viral populations, and more recently using ultradeep sequencing techniques and allow for adaptive variability and adaption to host-cells of different origins.

The 5' and 3' UTRs often play a critical role in the replication of RNA viruses. Albariño et al. (2003) showed that cis-acting elements within the last 108 nucleotides at the 3'end of FHV-RNA1 play a critical role for RNA1 replication, although, the exact sequence requirements were not determined. When using three different algoritms for the prediction of secondary RNA structures, Rosskopf identified (in his thesis) a short stretch of nucleotides which were predicted to form a stem-loop structure. A similar structure important for replication has been also identified also in the 3' end of RNA2. As a matter of fact, when deleting this stretch of nucleotides, the replication of RNA1 and RNA3 was severely hampered indicating an important role of this region for RNA replication [thesis Rosskopf].

Rosskopf (ibid), using plasmids and yeast to study the RNA *in situ,* examined the 3' UTR of NoV RNA1 for these elements based on the facts that viral UTR's have been shown to be important for Nodaviral RNA replication, previous work with *Flock House virus* (FHV) has shown that replication signals for both FHV RNA1 and RNA2 lie in their 3'UTRs, and that a stem-loop in the NoV RNA2 3'UTR was shown to be essential for its replication [Rosskopf JJ, Upton JH 3rd, Rodarte L, Romero TA, Leung MY, Taufer M, Johnson KL. A 3' terminal stem-loop structure in Nodamura virus RNA2 forms an essential cis-acting signal for RNA replication. Virus Res. 2010 Jun;150(1-2):12-21. Unsurprisingly, also the RNA1 seems to contain an essential element in the 3' UTR of RNA1 (nt 3162-3177); when mutating this sequence, a severe reduction in replication of RNA1 and generation of RNA3 was observed [thesis Rosskopf], To facilitate the study of RNA1 template properties and to identify cis-acting elements in the RNA1 without affecting the viral RdRp ORF, Rosskopf separated the mRNA and template functions of RNA1 onto two different molecules: an mRNA that expresses RdRp but cannot itself be replicated by said RdRp and an RNA1 template that doesn't produce RdRp but can be replicated *in trans* by the expressed RdRp. Insertion of GFP resulted in detetion of nt19-358 from RNA1 and abolished translation of RdRp. The nucleotides 1-19 and 359-3204 remained unchanged. 3 other deletion mutants were generated; ΔSalI (nt 1549-2256), ΔEagI (nt 1212-2564), and ΔXhoI (nt 2587-3041).

The mRNA construct has a GFP in RNA1 included (position 19 at the natural start codon) and they claim to keep the replicase inactive in that way and do not show expression of the protein. However, likely due to remaining plasmid-cryptic-promotor-derived RNA template replication activity of the GFP-RdRp mRNA constructs, the results of this study were not conclusive [thesis Rosskopf].

There have been attempts to generate a functional replicon based on the RNA1 of NoV [Biddlecome A, Habte HH, McGrath KM, Sambanthamoorthy S, Wurm M, Sykora MM, et al. (2019) Delivery of self-amplifying RNA vaccines in in vitro reconstituted virus-like particles. PLoS ONE 14(6): e0215031]. Biddlecome et al dispense RNA2 and add a gene of interest (GOI) to the 3' end of RNA1 partially deleting the genomic information encoding B1 and B2 protein . Biddlecome et al pose that any GOI can be coupled to the RNA dependent RNA polymerase (RdRp), in this single replicon system, as long as its length is below 1000 nt. Such a GOI is linked via a T2A cleavage peptide to the RdRp so the target antigen is separated from the replicase upon translation. Using their single replicon system in-cis strategy the ORF encoding the protein B2 which suppressess RNA interference by the host cell is not functional anymore. In consequence, the innate immune system of the host cell is activated after administration and replication efficacy of this replicon system is low.

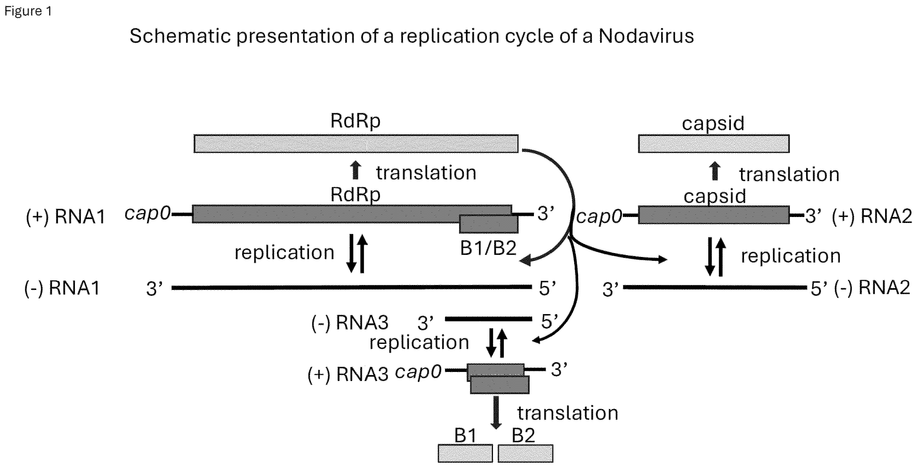

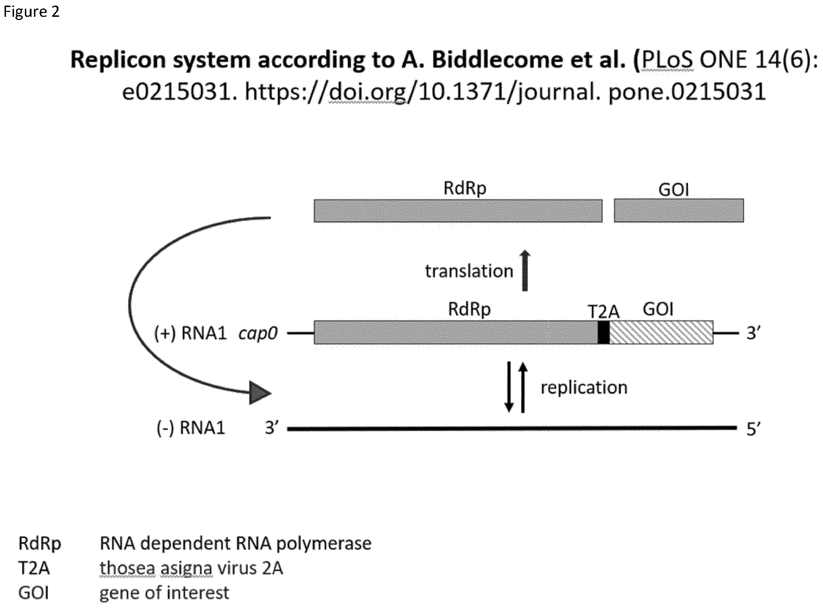

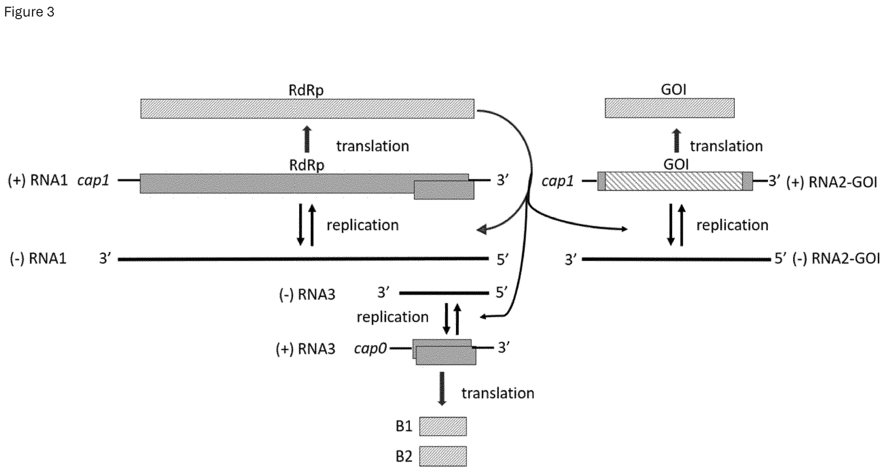

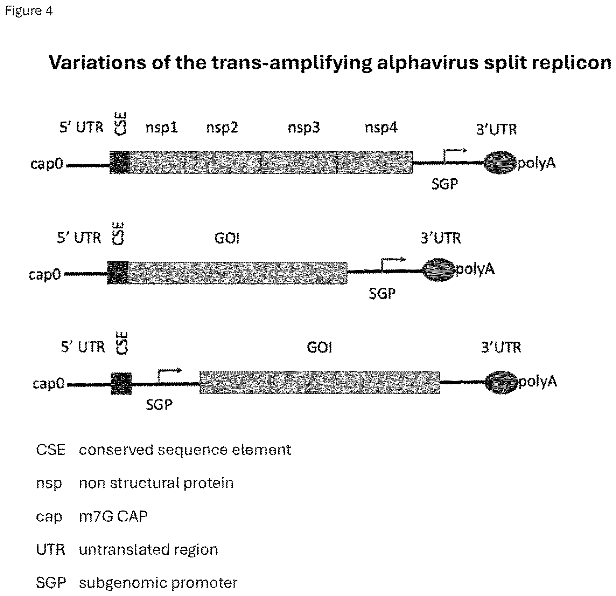

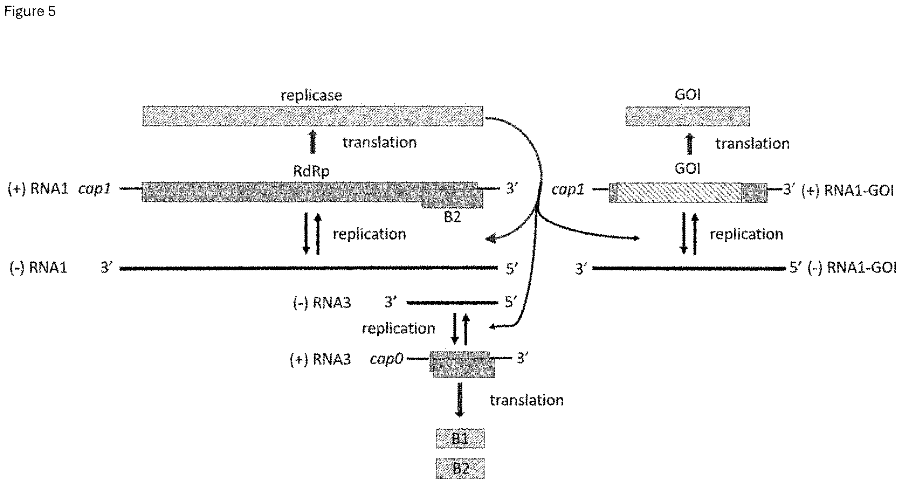

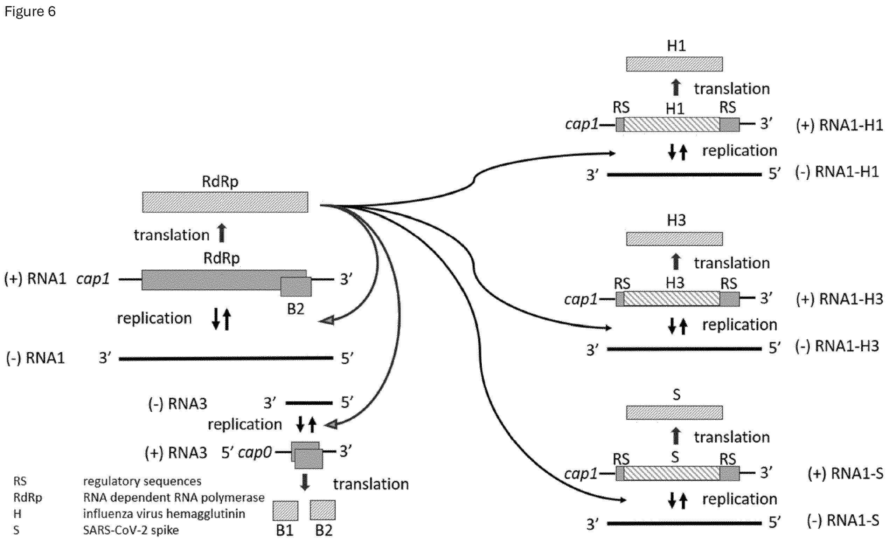

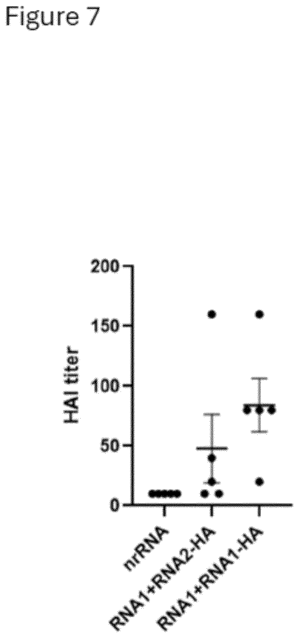

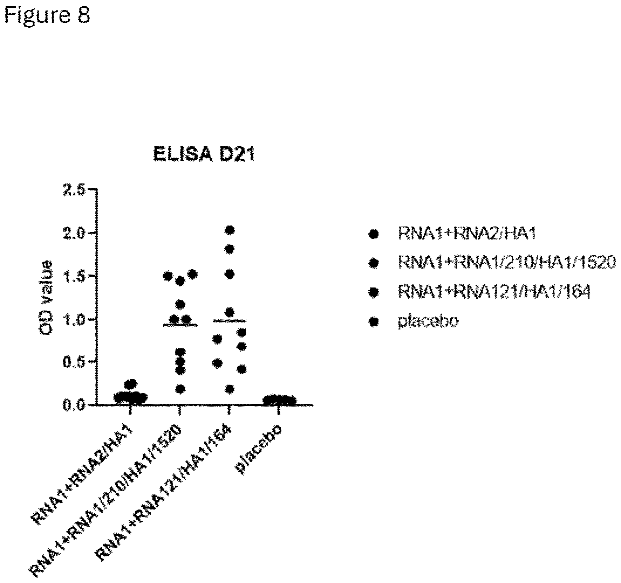

In summary, replicating RNA, including self-amplifying RNA (saRNA) and trans-amplifying RNA (taRNA) derived from positive strand RNA virus, holds a distinct potential for advancing the next generation of RNA-based vaccines. Unlike *in vitro* transcribed mRNA found in most current RNA vaccines, saRNA or taRNA can be massively replicated within cells in the presence of RNA-amplifying enzymes known as replicases. It was demonstrated that this property could enhance immune responses with reduced injected RNA amounts. In saRNA-based vaccines, replicase and antigens are encoded on the same mRNA molecule, resulting in very long RNA sequences, which poses significant challenges in production, delivery, and stability. Most recent (Yildiz A, R 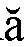 ileanu C, Beissert T. Trans-Amplifying RNA: A Journey from Alphavirus Research to Future Vaccines. Viruses. 2024 Mar 25; 16(4):503; see also figure 1 therein), Yildiz et al show that taRNA is a split derivative of saRNA that is composed of two RNAs: comprising a non-replicating mRNA (nrRNA) encoding the replicase and a so called transreplicon (TR) encoding the GOI, preferably a therapeutic transgene, that is multiplied by the replicase *in trans.*

### The invention

To further minimalize RNA-load upon therapeutic application, such as vaccination, in with particular of vaccination with multivalent vaccines, the invention provides an alternative split replicon system essentially and advantageously different from the split approach as discussed by Yildiz et al (ibid). The system according to the invention as disclosed herein is composed of two RNAs, each derived from a positive strand RNA virus: comprising a herein so called cisreplicon (CR, or cis-amplifying (ca)RNA) encoding the replicase, that upon entry in the cell is multiplied (self-amplified) by said replicase *in cis,* and a so called transreplicon (TR) encoding the GOI, preferably a therapeutic transgene, that is multiplied by said replicase *in trans.* In a preferred embodiment, and again essentially different from the split approach proposed by Yildiz et al (ibid), said GOI is not inserted in the viral genomic region that used to encode structural proteins (i.e. with alphavirus in the region downstream the initial location of the SGP) but instead inserted in the viral genomic region that used to encode non-structural proteins, in particular in the region that used to encode replicase proteins (i.e. with alphavirus in the region upstream the location of the initially present SGP). In a much preferred embodiment of the invention, said cisreplicon as well as said transreplicon share essentially the same molecular chassis derived from said positive strand virus, with all regulatory sequences and modifications required for the replication, transcription as well as translation of replicase-encoding-cisreplicon as well as the replication, transcription as well as translation of transgene-encoding-transreplicon essentially the same 5' - and 3'- regulatory sequences and modifications of regulatory sequences such as selected from the group of 5'-cap and cap modifications thereof, 5'- and 3'-conserved sequence elements (CSE) and modifications thereof, 5'- and 3'-untranslated regions (UTRs) and modifications thereof, 5'- and 3'- replication recognition sequences (RRS) and modifications thereof, 5'- and 3'- stem loop structures (SL) and modifications thereof as known in the art. This relative sameness of the molecular chassis and architecture of CR as well as multiple TRs not only facilitates ease and speed of construction, allowing plug-and-play characteristics to production of the system wherein pertinent replicase-encoding nucleic acid is simply replaced by deletion and subsequent insertion of one or more GOI-encoding nucleic acid, and wherein pertinent GOI-encoding nucleic acid is again simply replaced by deletion and subsequent insertion of yet other one or more GOI-encoding nucleic acid. This plug-and-play quality of the system here provided is particularly attractive when in need of rapidly developing anti-cancer vaccination for individual patients.

This relative sameness of the molecular chassis and architecture of CR as well as multiple TRs also provides essentially the same replication and translation characteristics to the CR as well as to different TR(s) in the cell, thereby bypassing otherwise present prioritizing routines of said parent positive strand RNA virus that may prioritize structural protein translation over replicase translation upon infection; the replicase often derived from genomic RNA and structural proteins often derived from subgenomic RNA, and with said bypassing allowing rapid stimulating of the cells of a vaccinated subject with the required presence and presentation of GOI-derived immunogens, without cells being underprovided with replicase. Typically, and not wishing to be bound by theory, when multivalency of the split system as provided here increases, and CR as well as TR are provided in equal amounts and 2 or more GOI are expressed from their respective TRs, the ratio of replicase expression from CR versus GOI-expression from TR decreases, whereas GOI expressed from a subgenomic area do not have said advantage.By tweaking or varying amounts of RNA per respective protein used, (typically ratio's of CR vs. TR from 1:10 to 10:1 may be used) the system as provided herein is thus allowing a substantial benefit in expression and presenting immunogens versus presenting replicase, a quality of the split system as provided herein that is not only beneficial in combatting infectious disease by vaccination with infectious agent immunogens providing protection against disease caused by infectious agents but typically also beneficial in combatting cancer when acceleration of vaccination means and methods with so called multi-epitope and/or neo-epitope immunogens that would induce immunity directed against tumours is typically required.

Typically, vaccine RNA-replicons are used in an RNA-vaccine. Target pharmacologically active or therapeutical genes-of-interest (GOI) for inclusion in a vaccine RNA-replicon are preferably selected from nucleic acids encoding a biologically or pharmacologically active nucleic acid, protein, or peptide. The desired or established GOI-target sequence will typically be selected, optimized, synthesized, and inserted into a DNA plasmid to use as a template for RNA-replicon synthesis, generally using *in vitro* transcription (IVT) that will be used to produce the RNA-replicon. Subsequently, the RNA-replicon, or parts thereof, when provided to a human or animal subject, host, or patient, replicates, preferably self-replicates, in cells of said subject to express the desired pharmacologically active or therapeutic nucleic acid, protein, or peptide product with its known therapeutic function. Preferably such a subject is a vertebrate such as a fish, a reptile, a bird, or a mammal, in particular a human. In another mode, such a subject may be a non-vertebrate such as a shrimp or a prawn.

The invention embraces an advantageous split replicon system allowing easy, plug-and-play, multivalency of RNA-therapeutics as well as RNA-vaccines that is fundamentally different from any of replicon systems as discussed above. The system as provided herein comprises combining essentially purified RNA derived from a positive strand RNA virus and encoding self-amplifying or self-replicating RdRp in a split-replicon fashion with one or more essentially purified RNAs having a GOI, or different GOIs inserted in the area of the RdRp-gene(s), and preferably structurally deleting open-reading-frames of viral structural proteins by avoiding use of nucleic acid encoding said structural protein(s) all together, or (for that matter) avoiding use of nucleic acid encoding a transgene (be it relevant or irrelevant) that is located in the area formerly used to encode said structural protein, and therewith rendering the system small, versatile, safe and propagation defective. The RdRp as well as the GOIs thus provided by said split system in a cell of a target subject favour transcription as well as productive translation of the RdRp-genes *in cis* as well as of the GOI-genes *in trans.* In a preferred embodiment, said different GOIs encode different protective antigens useful in a multivalent vaccine. Contrary to the old adage that GOI's are inserted at viral gene areas or open-reading-frames where once structural protein(s) were encoded, the invention now discloses to insert GOI's in viral gene areas or open reading frames where the RdRp genes typically were encoded. This not only simplifies construction by using the here provided saRdRp construct also as a template for GOI-insertion, thereby simplifying and allowing dual use of the molecular chassis, but also allows relying on RdRp-gene flanking virus-specific regulatory sequences such as terminal and subgenomic UTRs and other conserved or regulatory sequence elements (CSEs) that typically are used by the endogenous parent virus to prioritize RpRd expression over structural protein expression, but now are employed to prioritize GOI-expression as well. The invention provides a first replicon (cisreplicon, CR) encompassing a positive sense self-amplifying RdRp-gene allowing RdRp-amplification *in cis* and further provides one or more positive and/or negative sense GOI-replicons (transreplicon, TR), allowing RdRp-driven and advantageously multivalent GOI-replication *in trans.* In particular, the invention also provides a split replicon system wherein said CR and TR are combined and act in conjunction in a cell, allowing RdRp-driven and advantageously multivalent GOI-replication *in trans.*

The invention also provides split replicon system wherein said CR comprises SEQ ID NO: 1 and said TR comprises SEQ ID NO: 2. The invention also provides a split replicon system wherein said CR comprises SEQ ID NO: 1 and said TR comprises SEQ ID NO: 3. The invention also provides a split replicon system wherein said CR comprises SEQ ID NO: 1 and said TR comprises SEQ ID NO: 4. The invention also provides a split replicon system wherein said CR comprises SEQ ID NO: 1 and said TR comprises SEQ ID NO: 5. The invention also provides a split replicon system wherein said CR comprises SEQ ID NO: 7 and said TR comprises SEQ ID NO: 8. The invention also provides a split replicon system wherein said CR is combined with several TRs. The invention also provides a split replicon system wherein said CR comprises SEQ ID NO: 1 and is combined with several TRs that respectively comprise SEQ ID NO: 3 and SEQ ID NO: 4. The invention also provides a split replicon system wherein said CR comprises SEQ ID NO: 1 and is combined with several TRs that respectively comprise SEQ ID NO: 3 and SEQ ID NO: 5. The invention also provides a split replicon system wherein said CR comprises SEQ ID NO: 1 and is combined with several TRs that respectively comprise SEQ ID NO: 4 and SEQ ID NO: 5. The invention also provides a split replicon system wherein said CR comprises SEQ ID NO: 1 and is combined with several TRs that respectively comprise SEQ ID NO: 3, SEQ ID NO: 4 and SEQ ID NO: 5.

The invention also provides deoxyribonucleic acid (abbreviated DNA) encoding a CT or a TR according to the invention, preferably said DNA also provided with a promotor, such as a T7 or SP6 promotor, to provide for in vitro translation and production of said CR and TR RNAs. The invention also provides such a DNA encoding any of SEQ ID NO: 1, 2, 3, 4, 5, 7 or 8. The invention also provides DNA (herein also identified plug-and-play DNA) that is prepared for further insertion of a desired transgene (GOI nucleic acid) in a TR according to the invention. The invention also provides a DNA encoding at least 20 nucleotides (nt), preferably at least 40 nt, preferably at least 80 nt, of a 5'- or 3'-terminal regulatory sequence of RNA1 of a nodavirus without encoding a functional RNA-dependent RNA polymerase (RdRp)-gene derived from said virus. It is preferred that said 5'-terminal sequence is N-terminally covalently linked to nucleic acid encoding a transgene, it is preferred that said 5'-terminal sequence comprises SEQ ID NO: 18. It is preferred that said 3'-terminal sequence is C-terminally covalently linked to nucleic acid encoding a transgene, it is preferred that said 3'-terminal sequence comprises SEQ ID NO: 19. Said transgene may for example comprise one or more neoantigen epitopes, for example those identified as SEQ ID NO: 11, SEQ ID NO: 12, SEQ ID NO: 13, SEQ ID NO: 14, SEQ ID NO: 15, SEQ ID NO: 16 and SEQ ID NO: 17 optionally linked by a self-cleaving peptide such as T2A-peptide.

The invention also provides a DNA encoding at least 100 nucleotides (nt), preferably at least 150 nt, preferably at least 250 nt, of a 5'- or 3'-terminal regulatory sequence of RNA of an alphavirus without encoding a functional RNA-dependent RNA polymerase (RdRp)-gene derived from said virus. It is preferred that said 5'-terminal sequence is N-terminally covalently linked to nucleic acid encoding a transgene, it is preferred that said 5'-terminal sequence comprises SEQ ID NO: 20. It is preferred that said 3'-terminal sequence is C-terminally covalently linked to nucleic acid encoding a transgene, it is preferred that said 3'-terminal sequence comprises SEQ ID NO: 21. Said transgene may for example comprise one or more neoantigen epitopes, for example those identified as SEQ ID NO: 11, SEQ ID NO: 12, SEQ ID NO: 13, SEQ ID NO: 14, SEQ ID NO: 15, SEQ ID NO: 16 and SEQ ID NO: 17, optionally linked by a self-cleaving peptide such as T2A-peptide.

In a preferred embodiment, the invention discloses a pharmaceutical formulation comprising ribonucleic acid (RNA) derived from a distinct virus selected from any of the group of positive strand RNA-viruses, said formulation comprising a so-called split replicon set of at least two essentially purified ribonucleic acids (RNAs), said RNAs each having been provided with an 5 '- and a 3' untranslated region (UTR), preferably having been provided with 5'- and 3' replication recognition sequences (RRS) derived from said distinct virus and from which said each RNAs viral structural protein gene(s) of said virus have at least functionally been deleted, preferably structurally been deleted, more preferably been fully removed, to block and avoid (possibly infectious) viral particle formation and render the replicon propagation defective wherein a first positive sense RNA, herein identified as self-amplifying (sa)RdRp-RNA, encodes a functional RNA-dependent RNApolymerase (RdRp) -gene derived from said distinct virus and allows self-amplification *in cis* by said RdRp derived from said first RNA, and wherein at least a second (positive or negative sense ) RNA, herein identified as GOI#1-RNA and essentially partly derivable from the first RNA from which at least a part of the RdRP-gene is functionally deleted, preferably structurally deleted, to deprive said second RNA of functional RdRp-activity, and wherein said RdRp-gene part instead is at least partly replaced with a ribonucleic acid encoding a first gene-of interest (GOI) at the RdRp-gene location, said GOI#1-RNA allowing amplification of said GOI#1 *in trans* by said RdRp derived from said first RNA. The formulation according to the invention may advantageously comprise at least a third essentially purified RNA, herein identified as GOI#2-RNA, essentially derivable from the first RNA wherein said RdRp-gene is at least partly replaced with a ribonucleic acid encoding a second gene-of interest, allowing amplification of said GOI#1 and GOI#2 *in trans.* The formulation according may advantageously comprise at least a fourth essentially purified RNA, herein identified as GOI#3-RNA, essentially derivable from the first RNA wherein said RdPp-gene is at least partly replaced with a ribonucleic acid encoding a third gene-of interest, allowing amplification of said GOI#1, GOI#2 and GOI#3 *in trans,* and can be further extended to encompass amplification of GOI#4, 5 and 6. Said GOI's in themselves may also be encoding multi-epitopes or multi-polypeptides.

In a further embodiment, this new self-amplifying split replicon system is based on an RNA1 sequence of an Alphanodavirus which belongs to the family *Nodaviridae,* preferably the Nodamura virus (NoV), preferably strain MAG115 (gene bank ID AF174533). The system here provided is a split replicon system; one RNA segment encodes the wild type RNA1 from NoV MAG115, the second RNA fragment is assembled by a 210 nt long stretch derived from the 5' end of said RNA1. Nucleotide nt23 has been modified T→G to remove the start codon for the RdRp. After the 210 nt a AAC sequence has been introduced to generate a Kozak sequence and to ensure optimal protein translation of the GOI. The GOI is followed by a stretch of 1192 nt derived from RNA1 (nt 358-1549) and the last 164 nt also from RNA1 and preferably comprising at least a part of the RNA1-specific 3' UTR. Optionally a HDV ribozyme following the end of the 3' UTR provides monodisperse RNA transcripts. The split replicon system as provided is still able to produce protein B2 and thus, is able to suppress RNA interference. An even smaller replicon system can be envisioned which is set up by the wild type of RNA1 and a RNA1 segment encoding the GOI plus the stretches of nucleotides at its 5' and 3' ends which are important for recognition and binding of the template RNA by the replicase. In the case of NoV those are the first 21 nt from RNA1 followed by the start codon of the GOI, followed by the last 42 nt, preferably the last 164 nt from the 3' end of RNA1. According to Rosskopf (thesis) nucleotide 3162-3177 are forming a stem-loop structure which is described to be important for replication activity of RNA1.

### Legends

### Figure 1

General replication strategy of wild-type (wt) nodaviruses.

RNA1 encodes the RNA-dependent RNA polymerase (RdRp), which replicates the genomic and the subgenomic RNA segments via negative strand RNA intermediates. RNA2 encodes the viral capsid protein (CP) and is dispensable for RNA1 replication. During RNA1 replication a subgenomic RNA3 from RNA1 is synthesized overlapping with the 3' end of RNA1. RNA3 encodes 2 viral proteins, B1 and B2. B2 is suppressing RNA interference (RNAi). The function of B1 is still somewhat unclear and may add to suppressing innate immunity. In addition, RNA3 reportedly has been shown to be required for RNA2 replication.

### Figure 2

### Single replicon system. (Biddlecom's Cis-replicon system based on Nodaviral RNA1 having a GOI-inserted)

The gene of interest (GOI) is attached to the 3' end of the RNA1 via a T2A self-cleaving peptide so it is separated from the RdRp upon translation; the sequence encoding the B1 and B2 protein is partially deleted in that way. With this approach the replicase protein retains its biological activity and amplifies the RNA1. The GOI is launched from the same RNA1 fragment as the replicase (in-cis). The capsid protein from Cowpea Chlorotic Mottle Virus (CCMV) is used to wrap up the RNA1 in virus like particles (VLPs). The 3' end is followed by a ribozyme cleavage site to ensure uniform 3'ends. This approach is reportedly limited to approx. 1000 nt coding sequence for the GOI [Biddlecome A, Habte HH, McGrath KM, Sambanthamoorthy S, Wurm M, Sykora MM, et al. (2019) Delivery of self-amplifying RNA vaccines in in vitro reconstituted virus-like particles. PLoS ONE 14(6): e0215031. RdRp = RNA dependent RNA polymerase, T2A = thosea asigna virus 2A self-cleaving peptide, GOI = gene of interest.

### Figure 3

Earlier provided nodaviral RNA1 and RNA2 replicon system.

RNA1 encodes the RNA-dependent RNA polymerase (RdRp), which replicates the genomic RNA 1 and RNA2 and the subgenomic RNA3 segments via negative strand RNA intermediates. RNA2 encoding the nodaviral capsid protein is largely replaced by the gene of interest. The first 55 nt at the 5' end and the last 270 nt at the 3' end are derived from RNA2. The cap 0 structure at the 5' end of RNA1 and RNA2 of wt NoV is replaced by a cap 1 structure.

### Figure 4

Here provided variations of trans-amplifying alphavirus split replicon systems.

Alphaviruses contain non-segmented, single stranded RNA genomes of 11-12 kb, with a type 0 cap (N7mGppp) at the 5' end and a poly(A) tail at the 3' end. These characteristics make alphavirus genomes appear to the host cell as messenger RNA (mRNA) for immediate translation upon entry into the cytoplasm. There are two open reading frames (ORFs) in alphavirus genomes, encoding nonstructural and structural polyproteins, respectively. Flanking the ORFs are UTRs located at the 5' and 3' ends of the genome, as well as between the ORFs (the subgenomic 5' UTR with a subgenomic promotor, SGP). Both the 5' UTR and its complement in the 3' UTR of the minus-strand RNA comprise parts of the promoters recognized by the alphavirus replication complex, including the conserved sequence element (among which a so-called 51 base CSE and distinct alphavirus equivalent regulatory elements in the gene encoding the nonstructural protein 1 (nsp1).

Top: Schematic description of a self-amplifying-alphavirus derived RdRp RNA replicon here provided with a 5' regulatory element comprising virus-specific 5' replication recognition sequences (5'-RRS) such as the 5' UTR and the required conserved sequence elements (CSE) in the N-terminal part of NSP1, as well as virus-specific subgenomic- or 3'-replication recognition sequences such as the SGP as well as the 3'-UTR and polyA-tail (3'RRS) The ORF encoding the structural proteins is largely deleted. 5'-RRS, SGP as well as 3'-RRS may be aptly modified to remove expression of remnant NSP1 N-terminal fragments and/or to remove expression of remnant NSP4 C-terminal fragments or terminally stabilized by modifying an 5'RRS and/or an SGP and/or an 3'RRS as for example aptly explained in WO2008156829 and/or WO2017162266A1 and/or WO2017059902

Middle: Schematic description of trans-amplifying-alphavirus derived RdRp RNA replicon here provided with a T→G mutation in position 46 and a deletion in the ORF encoding the replicase wherein the gene-of-interest (GOI) is inserted contrary to the adage of earlier art before the SGP. The ORF encoding the structural proteins is deleted.

Combining the top construct with the middle construct, optionally in a pharmaceutical formulation, provides a split replicon system according to the invention wherein said system is comprising at least two essentially purified ribonucleic acids (RNAs) derived from an alphavirus from each of which RNA viral structural protein genes are at least functionally deleted, each provided with a 5' - and a 3' untranslated region (UTR) derived from said virus, preferably each provided with a 5' - and a 3' RRS derived from said virus, wherein a first positive sense RNA, herein identified as self-amplifying (sa)RdRp-RNA, encodes a functional RNA-dependent RNA polymerase (RdRp)-gene derived from said virus and allows self-amplification *in cis* by said RdRp derived from said first RNA, and wherein at least a second (positive or negative sense) RNA, herein identified as GOI#1-RNA essentially derivable from the first RNA wherein said RdPp-gene is at least partly replaced with a ribonucleic acid encoding a first gene-of interest (GOI#1) allows amplification of said GOI#1 *in trans* by said RdRp derived from said first RNA.

Bottom: Schematic description of trans-amplifying-alphavirus derived RNA here provided with a deletion in the ORF encoding the replicase located between the CSE and the SGP wherein the gene-of-interest (GOI) is inserted downstream from the CSE and SGP. Combining the top construct with the bottom construct provides a split replicon according to another invention wherein said system comprising at least two essentially purified ribonucleic acids (RNAs) derived from an alphavirus from each of which RNA viral structural protein genes are at least functionally deleted, each provided with a 5' - and a 3' untranslated region (UTR) derived from said virus, preferably each provided with a 5' - and a 3' RRS derived from said virus, wherein a first positive sense RNA, herein identified as self-amplifying (sa)RdRp-RNA, encodes a functional RNA-dependent RNA polymerase (RdRp)-gene derived from said virus and allows self-amplification *in cis* by said RdRp derived from said first RNA, and wherein at least a second (positive or negative sense) RNA, herein identified as GOI-RNA wherein a structural protein-gene is at least partly replaced with a ribonucleic acid encoding a gene-of interest and allows amplification of said GOI *in trans* by said RdRp derived from said first RNA.

### Figure 5

### Split replicon (here a trans-replicon system based on Nodaviral RNA1 is shown)

At least two different RNA1 molecules are co-transfected into a cell: (1: saRdRp-RNA) the entire wild type sequence of Nodaviral RNA1 encoding the replicase (RdRp) and protein B1 and B2 and (2) a one or more modified Nodaviral RNA1 molecule as follows: first 210 nt are derived from RNA1 followed by the GOI#1 or more GOIs (here an influenza vaccine antigen hemagglutin protein (HA1) is used), optionally followed by 1192 nt (358-1549), followed by the last 164 nt from the 3' end of RNA1; a ribozyme site may be added downstream to guarantee the correct sequence at the 3' end. The replicase is amplifying the RNA1 wild and the RNA-GOI#1 . During replication and amplification, the subgenomic RNA3 is generated which is used as template RNA for the expression of protein B2. This protein is reported to bind to both double-stranded RNA (dsRNA) and small interfering RNAs (siRNA), to prevent dicer processing of dsRNA and dicer activity and to sequester siRNA into RNA-induced silencing complex (RISC) to supress RNA interference (RNAi) [Sullivan CS, Ganem D. A virus-encoded inhibitor that blocks RNA interference in mammalian cells. J Virol. 2005 Jun;79(12):7371-9. doi: 10.1128/JVI.79.12.7371-7379.2005. PMID: 15919892; PMCID: PMC1143619]. Preferably, in the RNA1 based split replicon system the capO structure protecting the positive sense RNA1 is replaced by a cap1.

### Figure 6

Multivalent split RNA replicon system (trans-replicon system based on Nodaviral RNA1) with a schematic presentation of multivalent split replicon encoding the influenza virus hemagglutinin H1 and H3 and the SARS-CoV-2 spike protein.

Multiple different RNA1 molecules are co-transfected into a cell: (1: saRdRp-RNA) the entire wild type sequence of Nodaviral RNA1 encoding the replicase (RdRp) and protein B1 and B2 and (2) a one or more modified Nodaviral RNA1 molecule as follows: first 210 nt with regulatory sequences are derived from RNA1
- For RNA1-H1 respectively incorporating the GOI#1 (here an influenza vaccine antigen hemagglutinin protein (HA1) is used),
- For RNA1-H3 respectively incorporating the GOI#2 (here an influenza vaccine antigen hemagglutinin protein (HA3) is used), and
- For RNA1-S respectively incorporating the GOI#2 (here a COVID vaccine antigen hemagglutinin protein (SARS-Cov-2 spike protein S) is used),

Optionally all followed by 1192 nt (358-1549), all at least followed by the last 164 nt from the 3' end of RNA1; a ribozyme site is added downstream to guarantee the correct sequence at the 3' end. The replicase is amplifying the RNA1 wild type and also the RNA-GOI#1.

### Figure 7

Proof-of-principle results of mice -immunization with a pharmaceutical lipid nanoparticle formulation comprising a split replicon system as provided herein. Alternatively, a dendrimer formulation is provided. Dendrimers have emerged as an important group of nanostructured carriers for the development of nanomedicine to treat various diseases. Because of structural diversity and adaptability, dendrimers have been used to deliver drugs and genes in many different ways.

Groups of 6-8 weeks old female BALB/c mice were vaccinated intramuscularly twice either with a pharmaceutical formulation comprising
- SM102 as well as a split-replicon (SEQ ID NO: 1) comprising cis-replicating saRdRp (RNA1), and trans-replicating H1-RNA1 (SEQ ID NO: 3) derived from nodaviral RNA1 (1.5µg each RNA/dose)
- or SM102 as well as a split-replicon (SEQ ID NO: 1) comprising cis-replicating saRdRp (RNA1), and trans-replicating HA1-RNA (SEQ ID NO: 6) derived from nodaviral RNA2 (1.5µg each RNA/dose)
- or a non-replicating (nr) RNA-HA ((with SEQ ID NO: 10; 1.5µg RNA/dose),

Mice were vaccinated two times three weeks apart; all three GOI-carrying RNAs are encoding the prototype HA1 protein from influenza A virus (A/California/07/2009(H1N1)) which was sequenced optimized for expression in mammalian cells. Mice were bled on day 0 and on day 35 and serum was analyzed by an IgG ELISA specific for HA protein at a dilution of 1:6400 and tested in a hemagglutination inhibition assay (HAI). The mice immunized twice with RNA1+RNA1-HA showed high ELISA titer with a GMT slightly below 3 OD450, whereas mice immunized with the non-replicating mRNA encoding the HA didn't show any substantial IgG against the HA. The protective biological activity of the sera was tested by hemagglutination inhibition assay (HAI). The mice receiving two doses of the replicon RNA1+RNA1-HA showed a GMT of 80 whereas mice immunized twice with RNA1+RNA2-HA developed a GMT of 48. Animals receiving the non-replicating RNA1-HA had a GMT titer <10. HAI titers above 40 typically correlate with protection against a lethal challenge with a mouse-adapted influenza virus.

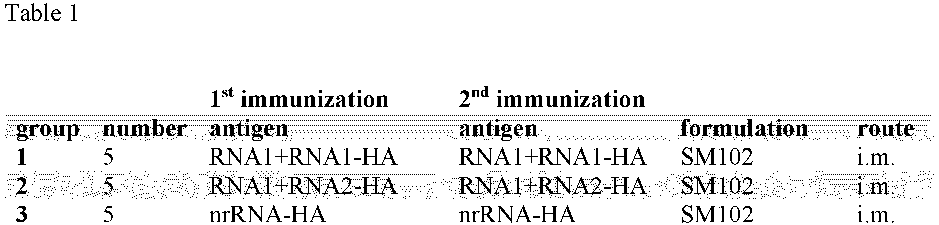

### Figure 8

Groups of 6-8 weeks old female BALB/c mice were vaccinated intramuscularly once either with a pharmaceutical formulation comprising
- SM102 as well as a split-replicon comprising cis-replicating saRdRp (SEQ ID NO: 1; RNA1), and trans-replicating GOI-RNA (SEQ ID NO: 2) derived from nodaviral RNA1 (1.5µg each RNA/dose)
- or SM102 as well as a split-replicon comprising cis-replicating saRdRp (SEQ ID NO:1; RNA1), and trans-replicating GOI-RNA (SEQ ID NO: 3) derived from nodaviral RNA1 (1.5µg each RNA/dose)
- or SM102 as well as a split-replicon comprising cis-replicating saRdRp (RNA1), and trans-replicating HA1-RNA (SEQ ID NO: 6) derived from nodaviral RNA2 (1.5µg each RNA/dose)
- or SM102 as negative control.

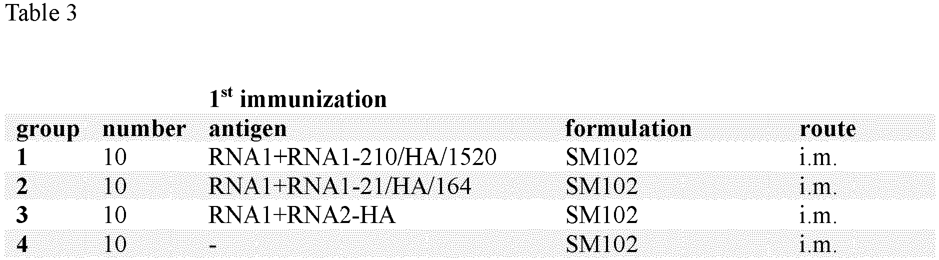

Mice were bled on day 21 and serum was analyzed by an IgG ELISA specific for HA protein at a dilution of 1:500. The mice immunized once with RNA1 (SEQ ID NO: 1) + RNA1-210/HA/1520 (SEQ ID NO: 3) and RNA1 (SEQ ID NO: 1) + RNA 1-21/HA/164 (SEQ ID NO: 2), respectively, showed similarly high ELISA titer with a GMT around an OD of 1, whereas mice immunized once with the RNA1 (SEQ ID NO: 1) + RNA2-HA (SEQ ID NO: 6) didn't show any substantial IgG titer against the HA in ELISA.

### Further detailed description

Currently used mRNA vaccines have some major limitations, including: (1) the relatively high vaccine doses needed to induce a robust immune response and (2) the need for repeated application (immune response boosting) and (3) unfavorable storage conditions. The need for high doses to achieve full efficacy results in substantial production capacity requirements and limits the co-administration/combination with other vaccines due to tolerability concerns. The need for repeated administration is also a sign of insufficient immune stimulation by the administered doses; however, the tolerability profile of very high doses may limit an escalation of the doses.

To overcome these issues, the use of self-amplifying RNA (saRNA), also called replicon technology, has been suggested instead of non-replicating mRNA. The saRNA can preserve the beneficial properties of mRNA vaccines, while requiring a substantially lower dose and overcoming the need for repeated administration. Research using saRNA systems have been shown to induce a protective immune response rapidly after a single dose, thus leading to fewer and lower doses and subsequently reducing the load on manufacturing at scale (Bloom K et al., Self-amplifying RNA vaccines for infectious diseases. Gene Ther. 2021;28:117-129).

To achieve this, saRNA is engineered from genomes encoding the replication machinery of naturally occurring RNA viruses, while replacing structural viral proteins with vaccine antigen(s). Importantly, the replicon represents only part of the replication machine of the virus, not a replication competent virus itself. After administration and upon uptake of the saRNA into the cytosol of a target host cell, the replicon directs the self-amplification of the gene(s) of interest (GOI) and produces large quantities of vaccine antigen, which is subsequently presented to the humoral and the cellular arm of the immune system.

As a result, a robust immune response is attained via the administration of a single dose of saRNA that is about 30 to 100 x lower than conventional non-replicating mRNA yet leads to a higher and longer expression of the vaccine antigen.

There are several viruses that are candidates for such a replicon system, all of them are based on RNA virus replication machines, most prominently alphaviruses, flaviviruses, measles virus and rhabdoviruses (Lundstrom K. Replicon RNA Viral Vectors as Vaccines. Vaccines. 2016;4:39). These replicon systems are relatively large and all genes encoding the replication machine as well as the GOI are combined on one segment and comes with a major disadvantage, in that the length of the construct is correlated with production cost due to the sheer amount of material needed and the decreased efficiency of in vitro transcription (IVT) with long constructs. For example, most of the replicon systems currently used are based on alphaviruses. For example, a shortcoming of the alphavirus replicon system is its large size since the replication machinery itself already requires approx. 8 kb and, depending on the nature of the antigen-encoding gene, the entire replicon RNA including the gene of interest comprises frequently about 9-13 kb. The size and complexity of this construct makes it difficult to generate high quality RNA with good yields and often requires complex and costly purification steps. For the purpose of multivalent vaccines the required alphavirus replication machinery may be too large altogether.

Essentially purified at least entails a near complete absence of double strand RNA in the system, preferably less than 2%, more preferably less than 1%, most preferably less than 0.5% is desired. The presence of double strand RNA can be tested with several methods known in the art (see for example J. Schönborn et al. Nucleic Acids Res.(1991)19, 2993. S. J. Richardson et al. J Clin Virol. (2010) 49(3); 180. F. Weber et al. J Virol (2006), 80(10):5059-64. K. Knoops et al. J Virol. (2012) 86(5); 2474. Or K. Karikó et al. Nucleic Acids Res. (2011) 39(21) e142), and furthermore essentially no DNA or protein contamination, preferably less than 1%, more preferably less than 0.5%, most preferably less than 0.1% of DNA or protein is desired, which can be tested with methods known in the art. Furthermore, it is desired that remnant DNA template levels are preferably < 200, more preferably < 100, most preferably < 50 ng/mg system RNA. A target system RNA purity of preferably > 80% free of impurities, more preferably > 90% free of impurities, most preferably > 95% of impurities is preferred. Throughout this application T may be used instead of U using the conventionality that T (thymidine) stands for U (uracil) in RNA. RNA stability and consequently antigen expression, critical to induce a robust immune response and vaccine efficacy, may be improved by including capping structures of the RNA at its 5' end, and modulating, preferably shortening the length of the poly(A) tail at its 3' end to <20 A, more preferably <15 A, more preferably <10 A, more preferably <5 A, more preferably to essentially no added poly(A) sequence. In addition, replacement of one or both pyrimidines (cytidine and uridine) with non-natural analogues (*i.e.,* 5-methylcytidine-5'-triphosphate (5mC) and pseudouridine-5'-triphosphate, respectively) reduces the stimulation of Toll-like receptors (TLR), protein kinase R (PKR) and retinoic acid-inducible protein I (RIG-I), resulting in a muted innate immune response *in vivo.* The use of modified nucleotides hides the exogenous RNA and can therefore significantly reduce this pattern recognition interaction and thus improve protein translation. Of note, RNA1 and RNA2 of Nodaviruses are 7-methylGpppN capped (cap-0 type) but are essentially not 3' polyadenylated which simplifies RNA manufacturing, allowing advantageous and straightforward in vivo, in vitro, as well as chemical, synthesis, and transcription of a system or formulation according to the invention. As reviewed by Yang et al., [Viruses. 2021 Jan 7; 13(1):73.], nodaviruses are small bisegmented RNA viruses belonging to the family *Nodaviridae.* Nodaviruses have been identified in different hosts, including insects, fishes, shrimps, prawns, pigs, dogs, and bats. A novel porcine nodavirus was first identified in the United States. RNA1 of the porcine nodavirus had the highest nucleotide identity (51.1%) to the Flock House virus, whereas its RNA2 gene shared the highest nucleotide identity (48%) with the RNA2 gene segment of caninovirus (Canine nodavirus). Genetic characterization classified porcine nodavirus as a new species under the genus *Alphanodavirus.* Within the *Nodaviridae,* traditionally the genus *Betanodavirus* is recognized as well, some also discern the genus *Gammanodavirus* as related to a bi-segmented virus of interest. Here all are included in the Nodaviruses.

The RNA1-HA (T7RNA1_210Cal09_1356Rz see sequence below) construct is under the control of a T7 promoter (last G of the promoter sequence is the first G of the 5' end of RNA1) and is generated as follows:
The 5' end of the RNA1-HA construct contains the first 210 nucleotides of Nodamura virus (NoV) RNA1 (strain MAG115) with a modification of nucleotide 23 T → G to remove the start codon for the RNA dependent RNA polymerase (RdRp). The 210 nucleotides are followed by AAC (nt 211-213) creating a Kozak sequence for optimal start of translation from the start codon ATG (nt 214-216) for the influenza A virus HA gene (the sequence is modified for optimal expression in humans). The HA gene is followed by a 1192 nt long stretch encoded by nt 358-1549 followed by the last 164 nt encoded by nucleotide 3041-3204 of the RNA1 MAG 115 sequence. To enable a correct processing of the 3' end a ribozyme site was introduced downstream of the 3' end of the RNA1-HA construct.

### Lipid nanoparticle formulation NanOZ LNP-DIY(SM102; OZ Biosciences)

Ready-to-use dried lipid mix at the total lipid concentration of 25 mM when reconstituted in 1mL Ethanol, for LNP-mRNA formulation.

LNPs represent the most effective and safe delivery systems for the translational success of nucleic acid drugs. LNPs are lipidic spherical vesicles formed by a combination of four main components: an ionizable cationic lipid, a helper phospholipid, cholesterol & a pegylated lipid, each having distinct functions. LNPs not only protect RNA from degradation, but also facilitate intracellular uptake and thus potentiate its efficacy. LNP/RNA systems self-assemble via electrostatic interactions between negatively charged RNA and ionizable cationic lipids.

A major advantage of the nodavirus-based split replicon system provided by the invention and disclosed herein is its small total genome size. Its total nucleotide number is at around 50 - 65% of which is minimally required for an alphavirus-derived split replicon system according to the invention. The nodaviral replicative RNA is encoding a single molecule uniting all functions important to replicate and amplify the Nodaviral RNA. RNA1 is encoding by only 3.2 kb nucleotides and is extremely easy to synthesise. In contrast the replicase complex of alphaviruses that is assembled by nsp1-4 and is encoded by >7kb of nt; due to their large genome size (10-12 kb), alpha- and flavivirus based replicon systems are very difficult to synthesise at reasonable quality. Moreover, the RNA of Nodaviruses doesn't contain a polyA tail, again reducing synthesis issues.

As said, RNA is increasingly difficult to synthesise at high quality with increasing lengths of the molecule. The second or further RNA segments encode on or more of the GOI. Since this is containing only the minimal segments from RNA1 to be recognized by the replicase as Nodavirus-derived RNA it is only marginally (21 nt on the 5'end and 42nt at its 3' end) bigger in size than the coding region of the GOI. A very useful advantage of the split replicon system as provided is the multiplexing of vaccines which can protect against different pathogens or different variants of a pathogen. The small saRdRp-RNA always stays the same and only one or more of the GOI-RNA segments is or are added. These characteristics make it the ideal platform technology for plug-and-play. The split replicon system provided herein has the advantage that the RNA1 encoding the RdRp is only 3.2 kb in lengths and combines all functions needed for a functional replicase in only 3.2 kb. The RNA fragment encoding the GOI is in the size range of the GOI plus the UTRs and is simple and cheap to manufacture. Moreover, the saRNA-replicase component of the system as disclosed is always the same, independent from the GOI which can be added as needed like in a building block system. It represents a truly modular system combining the positive characteristics of mRNA with its small size and ease of manufacturing. Multivalent mRNA vaccines will preferably be composed of three to five or more mRNA components, with a contemplated protective dose is e.g. 30 µg mRNA per antigen (like for COMIRNATY), the total mRNA will be 150 µg for 5 antigens. Due to the high amount of mRNA also more formulation material must be included in such a vaccine. This large dose of formulation will increase safety issues. RNA replicon technology as provided herein is projected be able to reduce the protective RNA dose to 1/10th or even 1/50th lower when comparing to non-replicating mRNA and thus, also the amount of formulation can be reduced accordingly, greatly reducing safety issues. Coming back to the example given above, a ten- to twenty-fold reduction will bring down an original 150 µg dose to 15 or even 3 µg RNA.

### Sequence listing

SEQ ID NO: 1
Nodamura virus RNA1 wild-type segment (GenBank: AF174533)
Position *1-21: 5 'UTR*
Position 22-3150: RdRp
Position *3151-3204: 3' UTR* 
GTATTGAATCCAAAACTCAAAATGCTGAACTACGAGACAATCATCAACGGCGCATCG
AGCGCTCTGAACATCGTTTCGCGTGCGTTAGGATACCGCGTGCCACTAGCCAAATC
GCTGGCGCTGGTCGCGGGTCCTGCGTGGTGTACAAAATAATCGTGCATCGACGC
ACGCTCGTGGCGTTCCTGGTAATCGGACCATACGCCACGGTGGTGCAGCACCGTCT
GCCGATGGCCCTTCAGAGGGCCATCATTGAATATACACGAGAAGACCGTGAGATCA
GCCTGTTTCCGCAAAATTCCATCGTTTCCGCAGAACACGCGCGGAAAGCGGATAAT
GGGCATCCGATCTCCGGGGGAACGCGTGATGTCGCGAGGGAGACTATTTCCCTTGC
CATAAGGGCCGCTGGTTTTCGTCATTACGAAATCAGCCCCGCGAGGCAATCACCAG
CTGAGGCGGCAAGCCACCAACATTATGCCGCCGCTGACCTCGTGAGAGCGGCTAC
TGAAGATAAGATCCAAGACGGTGATGTGGTAGTTGCCATCGACATCGATTACTACC
TGCGTGACATTGACCGCTACCTGGGTCGTGGTGTCCCGTTCATGGCTTACACCTTC
AATCCTGTTGAAGTAGCTGGCCGTGACGGTGACTCCTTTTTCCGGATCACGAACAA
TCAAGTCACGTTTGATGTTAGTGGCGGTGGATCTTGGTCCCATGAAGTTTGGGACT
GGTGCGCGTTTGGTGAGTTCATCGAGACCCGAGACGCGAGCTGGCTTGCTTGGTT
CGCCCGGGCGGTTGGACTCACCAAGTCGCAGATCCACAAAGTTCACTACTGCCGT
CCATGGCCGCAATCGCCCCATCGCGCTTTGGTGTGGTGTCTGCCTGTAGCAAGCTA
CTGGCGCTTCACTTTCATTCCGACGGACCTGCATACGCGCACGCTTCGGCGTGTGC
GTTATCAGGACACGTCCCGGCCCGGTTGGAATTCCATCGTCTCGACCGGGTCCGAA
GGCCTTAATATCAGCCTTGGTCGCGAAGGAGCTGATCATTGCGTGACGATTCCAAA
GGTGCACTACGACATGCTTATGGGTTTGTCGAGTGCGCAGTCGTTGTCGTCCCGCA
TGATCGGGCTCAAGTACACTGATCCTAGTGTACTCGCGACGGTTGCCCAATACTATC
AGGGCAAGAATGTTGAAGTTGCCGACGCTGACAGGATCGGCCGCGCCATAAATCC
CAAGGTCCACTGGCCAGCGCACGTCGAAGTTGACGAGGCGGAGGTTAGTGCTCG
GGTGTACGCCAGCCCGTTGGTATCTGACGAAAATATGATGCCTATGATCAAGCGCT
GGGAGACGCTGTCGTTGTCGCTGGACCGCCGGGTTACATTCCAACGTAATCCGAA
GGTTCCTGGAAAACGGCTCAGGGCTTATGCCATTGAGTTCGTTGACTTGGTTGTGC
CTGAGCGTGGTGTCGGAGTCCCCTATTCATTGGAGGACACCGCCGCCATGCTGGAC
 
AAACCAAGCCAGACCCTCGCCATCCAACAGGTGTGGAGACTGTCGACATGCCCC
CAAGAAGGCTCATCGAAGCGTTCGTGAAGAACGAACCGACCATGAAGGCTGGCC
GTATCATCTCGTCGTTCGCTGACATGCGGTTCCTACTGCGGTTTTCCAGCTATACGC
TGGCATTCCGTGATCAGGTGCTGCATGCAGAGCACAACCGGCATTGGTTTTGCCCG
GGTTTGACCCCCGAGCAGATCGCCACAAAAGTGGTTGATTACGTGTCCGGTGTTG
AAGAACCATCGGAGGGAGACTTTTCCAACTTTGATGGCACGGTTAGTGAGTGGCT
ACAACGCCACGTCATGAACGCCGTCTACCTGCGTTATTTCAATCACCGAGCGCAGC
GAGACCTCAGGTCGTATACCGACATGCTGGTCTCATGCCCCGCGAGGGCGAAGCG
ATTCGGTTTTGCTTATGACGCGGGTGTCGGCGTTAAGAGCGGGTCGCCAACAACTT
GCGACCTGAATACCGTGTGCAATGGTTTCCTCCAATATTGCTCCATTCGAATGACAC
ACCCAGAGCTGACACCAATCGATGCTTTCCGGCTCATCGGTCTCGCGTTTGGGGAC
GATTCCCTCTTCGAGCGACGTTTCGCTAAGAACTATGCGAAGGTTTCCGCCGAGGT
GGGGATGGTCCTCAAAATCGAGCGATTCGACCCGGCACAAGGCATCACTTTCCTC
GCCCGTGTTTATCCCGACCCCTACACGTCGACCACAAGTTTCCAGGACCCTTTGCG
TACCTGGAGGAAGCTCCACTTGACGACGCGCGATCCAACAATACCATTGGCAACG
GCTGCCATCGATCGCGTTGAGGGCTACCTCGTCACCGACGGCCTGAGCCCGCTTAC
TGGCGCGTATTGTCGCATGGTTAAGCGGGTTTACGAGGCCGGCGGAGCCGAGGAT
GCCGCCAAGAGGAGGTCGCGAAAATCCCATTCCCGCGAAAAGCCGTATTGGTTGA
CTGTTGGAGGCGCTTGGCCCCAAGATGTCAAGGACGTTGATCTTATGTTCCAGTGT
GCGGCCGCACGTACCGGAGTAGACCTCGAGACACTTCGGTCTCTGGATCAGCGTC
TAGGAGAAATCACTGACGTCTGGGCGGATATTACCATCAACCGGGATAATGAACCA
AACCCCTACAAGGATACACTGGACTTGGAGGGCCCGGCTGATGGCCGGGTGGACG
ATCGTGTATTTCAGAATGACAAACATGTCATGCGCTTACGAGCTAATCAAGTCACTT
CCAGCCAAGCTGGAGCAGCTGGCTCAGGAGACGCAAGCAACGATCCAAACGCTC
ATGATCGCGGATCCCAACGTCAACAAGGATCTGCGAGCGTTCTGCGAGTTCCTGAC
CGTGCAGCACCAGCGGGCGTATCGAGCGACGAACAGCCTGCTCATCAAACCGCGA
GTCGCAGCAGCGCTTCGCGGGGAGGAGCTGGACCTGGGCGAGGCGGACGTCGCC
GCCCGGGTCCGCCAGCTAAAACAACAGCTGGCGGAGCTCGAGATGGAAATCAAG
CCAGGGCACCAACAAGTGGCCCAAGTAAGCGGCAGGCGGAAGGCCGCAGCCGCA
GCTCCCGTGGCCCAGCTGGGTCGCGTGGGCGTGGTAAATGAGTGATTCATCGTCCC
ATCTGACGAAACCCGAACTAGGCTTATGCCAGTGGT
SEQ ID NO: 2
RNA1-21/HA1/164:
Position *1-21: 5'UTR*
Position 22-1725: GOI - HA from influenza A virus/California/07/09 (H1N1) optimized for expression in humans
Position 1726-1889: *Final 164 nt from RNA1 (nt 3041-3204) including the 3'UTR* 
GTATTGAATCCAAAACTCAAAATGAAGGCCATCCTGGTGGTGCTGCTGTACACCTTCG
CCACCGCCAACGCCGACACCCTGTGCATCGGCTACCACGCCAACAACAGCACCGA
CACCGTGGACACCGTGCTGGAGAAGAACGTGACCGTGACCCACAGCGTGAACCT
GCTGGAGGACAAGCACAACGGCAAGCTGTGCAAGCTGAGAGGCGTGGCCCCCCT
GCACCTGGGCAAGTGCAACATCGCCGGCTGGATCCTGGGCAACCCCGAGTGCGAG
AGCCTGAGCACCGCCAGCAGCTGGAGCTACATCGTGGAGACCCCCAGCAGCGAC
AACGGCACCTGCTACCCCGGCGACTTCATCGACTACGAGGAGCTGAGAGAGCAGC
TGAGCAGCGTGAGCAGCTTCGAGAGATTCGAGATCTTCCCCAAGACCAGCAGCTG
GCCCAACCACGACAGCAACAAGGGCGTGACCGCCGCCTGCCCCCACGCCGGCGC
CAAGAGCTTCTACAAGAACCTGATCTGGCTGGTGAAGAAGGGCAACAGCTACCCC
AAGCTGAGCAAGAGCTACATCAACGACAAGGGCAAGGAGGTGCTGGTGCTGTGG
GGCATCCACCACCCCAGCACCAGCGCCGACCAGCAGAGCCTGTACCAGAACGCC
GACGCCTACGTGTTCGTGGGCAGCAGCAGATACAGCAAGAAGTTCAAGCCCGAGA
TCGCCATCAGACCCAAGGTGAGAGACAGAGAGGGCAGAATGAACTACTACTGGA
CCCTGGTGGAGCCCGGCGACAAGATCACCTTCGAGGCCACCGGCAACCTGGTGGT
GCCCAGATACGCCTTCGCCATGGAGAGAAACGCCGGCAGCGGCATCATCATCAGC
GACACCCCCGTGCACGACTGCAACACCACCTGCCAGACCCCCAAGGGCGCCATCA
ACACCAGCCTGCCCTTCCAGAACATCCACCCCATCACCATCGGCAAGTGCCCCAA
GTACGTGAAGAGCACCAAGCTGAGACTGGCCACCGGCCTGAGAAACATCCCCAG
CATCCAGAGCAGAGGCCTGTTCGGCGCCATCGCCGGCTTCATCGAGGGCGGCTGG
ACCGGCATGGTGGACGGCTGGTACGGCTACCACCACCAGAACGAGCAGGGCAGC
GGCTACGCCGCCGACCTGAAGAGCACCCAGAACGCCATCGACGAGATCACCAAC
AAGGTGAACAGCGTGATCGAGAAGATGAACACCCAGTTCACCGCCGTGGGCAAG
GAGTTCAACCACCTGGAGAAGAGAATCGAGAACCTGAACAAGAAGGTGGACGAC
GGCTTCCTGGACATCTGGACCTACAACGCCGAGCTGCTGGTGCTGCTGGAGAACG
AGAGAACCCTGGACTACCACGACAGCAACGTGAAGAACCTGTACGAGAAGGTGA
GAAGCCAGCTGAAGAACAACGCCAAGGAGATCGGCAACGGCTGCTTCGAGTTCT
ACCACAAGTGCGACAACACCTGCATGGAGAGCGTGAAGAACGGCACCTACGACT
 
ACCCCAAGTACAGCGAGGAGGCCAAGCTGAACAGAGAGGAGATCGACGGCGTGA
AGCTGGAGAGCACCAGAATCTACCAGATCCTGGCCATCTACAGCACCGTGGCCAG
CAGCCTGGTGCTGGTGGTGAGCCTGGGCGCCATCAGCTTCTGGATGTGCAGCAAC
GGCAGCCTGCAGTGCAGAATCTGCATCTAATAACTCGAGATGGAAATCAAGCCAGGG
CACCAACAAGTGGCCCAAGTAAGCGGCAGGCGGAAGGCCGCAGCCGCAGCTCCCGTG
GCCCAGCTGGGTCGCGTGGGCGTGGTAAATGAGTGATTCATCGTCCCATCTGACGAAA
CCCGAACTAGGCTTATGCCAGTGGT
SEQ ID NO: 3
RNA1-210/HA1/1520:
Position *1-210: 5' UTR plus regulatory element*
*Position 23: T→G mutation to delete start codon*
Position *211-213: AAC inserted to get optimized Kozak sequence*
Position *214-1917:* GOI - HA from influenza A virus/California/07/09 (H1N1) optimized for expression in humans
Position 1918-3109: nt 359-1549 from RNA1
Position 3110-3273: last 164 nt from RNA1 
GTATTGAATCCAAAACTCAAAAGGCTGAACTACGAGACAATCATCAACGGCGCATCGAG
CGCTCTGAACATCGTTTCGCGTGCGTTAGGATACCGCGTGCCACTAGCCAAATCGCTGG
CGCTGGTCGCGGGGTCCTGCGTGGTGTACAAAATAATCGTGCATCGACGCACGCTCGT
GGCGTTCCTGGTAATCGGACCATACGCCACGGTGAACATGAAGGCCATCCTGGTGGT
GCTGCTGTACACCTTCGCCACCGCCAACGCCGACACCCTGTGCATCGGCTACCACG
CCAACAACAGCACCGACACCGTGGACACCGTGCTGGAGAAGAACGTGACCGTGA
CCCACAGCGTGAACCTGCTGGAGGACAAGCACAACGGCAAGCTGTGCAAGCTGA
GAGGCGTGGCCCCCCTGCACCTGGGCAAGTGCAACATCGCCGGCTGGATCCTGGG
CAACCCCGAGTGCGAGAGCCTGAGCACCGCCAGCAGCTGGAGCTACATCGTGGA
GACCCCCAGCAGCGACAACGGCACCTGCTACCCCGGCGACTTCATCGACTACGAG
GAGCTGAGAGAGCAGCTGAGCAGCGTGAGCAGCTTCGAGAGATTCGAGATCTTCC
CCAAGACCAGCAGCTGGCCCAACCACGACAGCAACAAGGGCGTGACCGCCGCCT
GCCCCCACGCCGGCGCCAAGAGCTTCTACAAGAACCTGATCTGGCTGGTGAAGAA
GGGCAACAGCTACCCCAAGCTGAGCAAGAGCTACATCAACGACAAGGGCAAGGA
GGTGCTGGTGCTGTGGGGCATCCACCACCCCAGCACCAGCGCCGACCAGCAGAGC
CTGTACCAGAACGCCGACGCCTACGTGTTCGTGGGCAGCAGCAGATACAGCAAGA
AGTTCAAGCCCGAGATCGCCATCAGACCCAAGGTGAGAGACAGAGAGGGCAGAA
TGAACTACTACTGGACCCTGGTGGAGCCCGGCGACAAGATCACCTTCGAGGCCAC
 
CGGCAACCTGGTGGTGCCCAGATACGCCTTCGCCATGGAGAGAAACGCCGGCAGC
GGCATCATCATCAGCGACACCCCCGTGCACGACTGCAACACCACCTGCCAGACCC
CCAAGGGCGCCATCAACACCAGCCTGCCCTTCCAGAACATCCACCCCATCACCATC
GGCAAGTGCCCCAAGTACGTGAAGAGCACCAAGCTGAGACTGGCCACCGGCCTG
AGAAACATCCCCAGCATCCAGAGCAGAGGCCTGTTCGGCGCCATCGCCGGCTTCA
TCGAGGGCGGCTGGACCGGCATGGTGGACGGCTGGTACGGCTACCACCACCAGAA
CGAGCAGGGCAGCGGCTACGCCGCCGACCTGAAGAGCACCCAGAACGCCATCGA
CGAGATCACCAACAAGGTGAACAGCGTGATCGAGAAGATGAACACCCAGTTCACC
GCCGTGGGCAAGGAGTTCAACCACCTGGAGAAGAGAATCGAGAACCTGAACAAG
AAGGTGGACGACGGCTTCCTGGACATCTGGACCTACAACGCCGAGCTGCTGGTGC
TGCTGGAGAACGAGAGAACCCTGGACTACCACGACAGCAACGTGAAGAACCTGT
ACGAGAAGGTGAGAAGCCAGCTGAAGAACAACGCCAAGGAGATCGGCAACGGCT
GCTTCGAGTTCTACCACAAGTGCGACAACACCTGCATGGAGAGCGTGAAGAACGG
CACCTACGACTACCCCAAGTACAGCGAGGAGGCCAAGCTGAACAGAGAGGAGAT
CGACGGCGTGAAGCTGGAGAGCACCAGAATCTACCAGATCCTGGCCATCTACAGC
ACCGTGGCCAGCAGCCTGGTGCTGGTGGTGAGCCTGGGCGCCATCAGCTTCTGGA
TGTGCAGCAACGGCAGCCTGCAGTGCAGAATCTGCATCTAATAAACGCGTGATGTC
GCGAGGGAGACTATTTCCCTTGCCATAAGGCCGCTGGTTTTCGTCATTACGAAATCAG
CCCCGCGAGGCAATCACCAGCTGAGGCGGCAAGCCACCAACATTATGCCGCCGCTGA
CCTCGTGAGAGCGGCTACTGAAGATAAGATCCAAGACGGTGATGTGGTAGTTGCCATCG
ACATCGATTACTACCTGCGTGACATTGACCGCTACCTGGGTCGTGGTGTCCCGTTCATG
GCTTACACCTTCAATCCTGTTGAAGTAGCTGGCCGTGACGGTGACTCCTTTTTCCGGAT
CACGAACAATCAAGTCACGTTTGATGTTAGTGGCGGTGGATCTTGGTCCCATGAAGTTT
GGGACTGGTGCGCGTTTGGTGAGTTCATCGAGACCCGAGACGCGAGCTGGCTTGCTT
GGTTCGCCCGGGCGGTTGGACTCACCAAGTCGCAGATCCACAAAGTTCACTACTGCCG
TCCATGGCCGCAATCGCCCATCGCGCTTTGGTGTGGTGTCTGCCTGTAGCAAGCTACT
GGCGCTTCACTTTCATTCCGACGGACCTGCATACGCGCACGCTTCGGCGTGTGCGTTAT
CAGGACACGTCCCGGCCCGGTTGGAATTCCATCGTCTCGACCGGGTCCGAAGGCCTTA
ATATCAGCCTTGGTCGCGAAGGAGCTGATCATTGCGTGACGATTCCAAAGGTGCACTAC
GACATGCTTATGGGTTTGTCGAGTGCGCAGTCGTTGTCGTCCCGCATGATCGGGCTCAA
GTACACTGATCCTAGTGTACTCGCGACGGTTGCCCAATACTATCAGGGCAAGAATGTTGA
AGTTGCCGACGCTGACAGGATCGGCCGCGCCATAAATCCCAAGGTCCACTGGCCAGC
GCACGTCGAAGTTGACGAGGCGGAGGTTAGTGCTCGGGTGTACGCCAGCCCGTTGGT
ATCTGACGAAAATATGATGCCTATGATCAAGCGCTGGAGACGCTGTCGTTGTCGCTGG
 
ACCGCCGGGTTACATTCCAACGTAATCCGAAGGTTCCTGGAAAACGGCTCAGGGCTTAT
GCCATTGAGTTCGTTGACTTGGTTGTGCCTGAGCGTGGTGTCGGAGTCCCCTATTCATT
GGAGGACACCGCCGCCATGCTGGACAAACCAAGCCAGACCCTCGCCATCCAACAGGT
GTGGGAGACTGCTCGAGATGGAAATCAAGCCAGGGCACCAACAAGTGGCCCAAGT
AAGCGGCAGGCGGAAGGCCGCAGCCGCAGCTCCCGTGGCCCAGCTGGGTCGCGT
GGGCGTGGTAAATGAGTGATTCATCGTCCCATCTGACGAAACCCGAACTAGGCTTA
TGCCAGTGGT
SEQ ID NO: 4
RNA1-21/HA3/164
Position *1-21: 5' UTR*
Position 22-1722: GOI - HA from influenza A virus/Massachusetts/18/2022 (H3N2)
Position 1723-1886: *Final 164 nt from RNA1 (nt 3041-3204) including the 3'UTR* 
GTATTGAATCCAAAACTCAAAATGAAGGCTATCATTGCTTTGAGCAACATTCTATGTCT
TGTTTTCGCTCAAAAAATACCTGGAAATGACAATAGCACGGCAACGCTGTGCCTTG
GGCACCATGCAGTACCAAACGGAACGATAGTGAAAACAATCACAAATGACCGAAT
TGAAGTTACTAATGCTACTGAGTTGGTTCAGAATTCATCAATAGGTAAAATATGCAA
CAGTCCTCATCAGATCCTTGATGGAGGGAACTGCACACTAATAGATGCTCTATTGG
GGGACCCTCAGTGTGACGGCTTTCAAAATAAGGAATGGGACCTTTTTGTTGAACG
AAGCAGAGCCAACAGCAGCTGTTACCCTTATGATGTGCCGGATTATGCCTCCCTTA
GGTCACTAGTTGCCTCATCCGGAACACTGGAATTTAAAAATGAAAGCTTCAATTGG
ACCGGAGTCAAACAAAACGGAACAAGTTCTGCGTGCAAAAGGGGATCTAGTAGT
AGTTTTTTAGTAGATTAAATTGGTTGACCAGCTTAAACAACATATATCCAGCACAG
AACGTGACTATGCCAAACAAGGAACAATTTGACAAATTGTACATTTGGGGGGTTCA
CCACCCGGATACGGACAAGAACCAATTCTCCCTGTTTGCTCAATCATCAGGAAGAA
TCACAGTATCTACCAAAAGAAGCCAACAAGCTGTAATCCCAAATATCGGATCTAGA
CCCAGAGTAAGGGATATCCCTAGCAGAATAAGCATCTATTGGACAATAGTAAAACC
GGGAGACATACTTTTGATTAACAGCACAGGGAATCTAATTGCTCCTAGGGGTTACT
TCAAAATACGAAGTGGGAAAAGCTCAATAATGAGATCAGACGCACCCATTGGCAA
ATGTAAGTCTGAATGCATCACTCCAAATGGAAGCATTCCCAATGACAAACCGTTCC
AAAATGTAAACAGGATCACATACGGGGCCTGTCCCAGATACGTTAAGCAAAGCAC
CCTGAAATTGGCAACAGGAATGCGAAATGTACCTGAGAAACAAACCAGAGGCATA
TTTGGTGCAATAGCGGGTTTCATAGAAAATGGATGGAGGGAATGGTGGATGGTTG
GTACGGTTTCAGGCATCAAAATTCTGAGGGAAGAGGACAAGCAGCAGATCTCAAA
AGCACTCAAGCAGCAATCGATCAAATCAGTGGGAAGCTGAATCGATTGATCGGAA
 
AAACCAACGAGAAATTCCATCAGATTGAAAAAGAATTCTCAGAAGTAGAAGGAAG
AGTTCAAGACCTCGAGAAATATGTTGAGGACACTAAAATAGATCTCTGGTCATACA
ACGCGGAGCTTCTTGTTGCCCTGGAGAACCAACATACGATTGACCTAACTGACTCA
GAAATGAACAAGCTGTTTGAAAAAACAAAGAAGCAACTGAGGGAAAATGCTGAG
GATATGGGAAATGGTTGTTTCAAAATATACCACAAATGTGACAATGCCTGCATAGG
ATCAATAAGAAATGAAACTTATGACCACAATGTGTACAGGGATGAAGCATTAAACA
ACCGGTTCCAGATCAAGGGAGTTGAGCTGAAGTCAGGGTACAAAGATTGGATCCT
ATGGATTTCCTTTGCCATGTCATGTTTTTGCTTTGTATTGCTTTGTTGGGGTTCATC
ATGTGGGCCTGCCAAAAGGGCAACATTAGATGCAACATTTGCATTTAACTCGAGAT
GGAAATCAAGCCAGGGCACCAACAAGTGGCCCAAGTAAGCGGCAGGCGGAAGGCCGC
AGCCGCAGCTCCCGTGGCCCAGCTGGGTCGCGTGGGCGTGGTAAATGAGTGATTCAT
CGTCCCATCTGACGAAACCCGAACTAGGCTTATGCCAGTGGT
SEQ ID NO: 5
RNA1-21/S/164
Position 1-21: 5' UTR
Position 22-3846: SARS-CoV-2 Spike Wuhan
Position 3847-4010: final 164nt from RNA1 wildtype 
GTATTGAATCCAAAACTCAAAATGTTCGTTTTCTTGGTGCTCCTGCCTCTGGTGTCCTC
CCAGTGCGTGAACCTGACCACTCGTACCCAGCTCCCACCTGCTTACACCAACAGC
TTTACTCGCGGCGTGTACTATCCCGACAAGGTGTTTCGTTCCAGCGTTCTGCATAGC
ACCCAGGACCTGTTTCTCCCTTTCTTTAGCAATGTCACCTGGTTCCACGCTATCCAC
GTGTCCGGCACCAATGGTACTAAGCGTTTTGATAATCCGGTGCTCCCATTCAACGA
CGGCGTGTATTTCGCTTCCACCGAGAAGTCCAACATTATCCGTGGCTGGATCTTCG
GCACAACCCTGGACTCTAAGACACAGAGTCTGCTCATCGTTAACAACGCCACCAA
TGTGGTGATCAAGGTCTGCGAGTTCCAATTTTGCAACGACCCGTTCCTGGGAGTGT
ACTACCACAAGAACAACAAGTCTTGGATGGAGAGCGAGTTCCGCGTCTACTCCTC
TGCCAACAACTGCACCTTCGAATACGTTTCCCAGCCCTTTCTGATGGACTTGGAGG
GGAAGCAAGGGAACTTCAAGAACCTGCGCGAGTTCGTCTTCAAGAATATCGACGG
CTACTTCAAGATCTACAGCAAGCATACCCCCATCAATCTCGTGAGAGACCTCCCCC
AGGGCTTCTCTGCTCTGGAGCCTTTGGTTGATTTGCCCATCGGTATCAACATCACTC
GCTTTCAGACACTGCTGGCCCTGCACCGCTCCTATCTCACCCCAGGTGATTCTAGC
AGTGGTTGGACTGCAGGAGCCGCGGCCTACTATGTGGGATACCTGCAGCCTCGTAC
CTTTCTCCTGAAGTACAATGAGAACGGCACAATTACTGATGCCGTGGACTGCGCAC
TGGATCCCTTGAGCGAGACCAAGTGCACATTGAAGTCCTTCACTGTGGAAAAAGG
 
CATCTACCAGACAAGCAATTTCAGAGTCCAGCCCACCGAGTCTATCGTTCGCTTTC
CGAACATCACCAACCTGTGCCCCTTCGGGGAAGTTTTAACGCCACTCGGTTTGCC
AGTGTCTATGCGTGGAACCGGAAAAGGATCAGCAACTGCGTGGCGGACTACAGCG
TTCTGTACAACTCCGCGTCTTTCTCTACCTTTAAATGCTACGGGGTCAGCCCTACAA
AACTGAACGATCTCTGTTTCACAAACGTGTATGCCGATAGCTTTGTGATTCGCGGA
GACGAAGTCCGGCAGATCGCCCCTGGACAAACAGGCAAGATCGCTGATTACAACT
ACAAGCTGCCCGACGACTTCACCGGCTGTGTGATTGCCTGGAACAGCAACAACCT
GGATTCTAAGGTCGGCGGCAACTACAACTATCTGTATAGGCTGTTTAGGAAGTCCA
ACCTCAAGCCTTTCGAAAGAGATATCAGTACTGAGATTTACCAGGCGGGCTCTACC
CCTTGTAATGGAGTCGAGGGCTTTAACTGTTACTTCCCCTTGCAGAGCTACGGTTT
CCAGCCTACTAACGGTGTGGGATATCAACCGTATCGCGTCGTGGTGTTGAGCTTTG
AATTGCTGCATGCTCCCGCCACCGTGTGTGGCCCTAAGAAGTCCACCAATTTGGTC
AAGAACAAGTGCGTCAACTTTAACTTCAACGGACTGACAGGCACCGGCGTGCTGA
CCGAATCCAACAAGAAATTCCTGCCGTTCCAGCAGTTCGGCAGGGATATTGCGGAT
ACCACCGACGCTGTGCGGGACCCTCAGACATTGGAGATCCTCGATATCACCCCCTG
CTCTTTTGGTGGAGTTTCTGTGATCACTCCTGGCACAAATACCTCTAATCAGGTTGC
CGTGCTGTACCAGGACGTCAACTGTACAGAGGTGCCCGTTGCGATCCATGCTGATC
AGCTCACTCCTACTTGGAGAGTCTATTCCACTGGTTCCAACGTGTTCCAGACCCGC
GCCGGATGTCTGATTGGCGCTGAACACGTGAATAATAGTTACGAGTGTGATATCCC
CATCGGCGCTGGAATTTGCGCTTCCTATCAGACCCAGACTAATTCTCCTCGTCGCGC
TAGGTCTGTGGCCTCTCAGTCTATCATCGCCTATACTATGAGCCTGGGAGCGGAGA
ATTCCGTCGCATACAGCAACAACTCCATCGCCATTCCAACCAACTTCACTATTAGCG
TGACTACCGAGATTCTGCCCGTGTCCATGACCAAGACCTCCGTGGACTGTACCATG
TACATTTGCGGGGACTCTACCGAATGTAGCAACCTCCTGCTCCAGTACGGTTCCTTT
TGCACTCAGTTGAACAGAGCCCTCACAGGCATTGCCGTCGAACAAGACAAGAAC
ACCCAAGAGGTGTTCGCGCAAGTTAAGCAGATCTACAAGACTCCGCCAATCAAAG
ACTTTGGGGGTTTCAATTTCAGTCAGATCCTGCCGGACCCGTCTAAACCATCTAAG
CGCTCTTTCATCGAGGACCTCCTGTTCAATAAGGTGACCCTGGCGGATGCCGGCTT
TATCAAACAGTACGGTGATTGTCTCGGCGATATTGCTGCACGCGACCTGATCTGTG
CCCAGAAGTTTAACGGTCTGACTGTCCTCCCACCACTCCTGACTGACGAGATGATC
GCCCAGTATACAAGCGCCCTGCTGGCTGGCACTATCACCTCTGGCTGGACTTTTGG
TGCCGGCGCTGCCTTGCAGATCCCCTTCGCTATGCAGATGGCCTACCGTTTTAATGG
GATTGGGGTCACTCAGAACGTGCTGTACGAGAACCAGAAACTCATCGCCAACCAG
TTCAACAGTGCCATTGGGAAAATCCAGGACAGCTTGTCTTCTACCGCCTCCGCCCT
 
GGGCAAACTGCAGGATGTCGTGAACCAAAACGCCCAGGCACTGAACACTCTGGTT
AAGCAACTGTCTTCCAATTTCGGCGCGATCTCTTCCGTGCTGAACGATATCCTCTCC
CGCTTGGACCCTCCCGAGGCTGAGGTGCAAATTGACCGTCTGATTACTGGGCGCCT
GCAATCTCTCCAGACCTATGTGACACAGCAGCTGATTAGAGCAGCTGAGATCCGCG
CCTCCGCTAATCTCGCTGCCACCAAAATGTCTGAGTGCGTCCTGGGACAGAGCAA
ACGCGTTGACTTCTGCGGGAAAGGCTATCACCTGATGAGTTTTCCCCAGTCCGCAC
CCCACGGCGTGGTTTTCCTCCATGTGACTTACGTGCCTGCTCAGGAAAAGAACTTC
ACCACCGCTCCAGCCATCTGTCACGATGGGAAGGCTCATTTCCTAGGGAAGGTGT
GTTCGTTTCTAACGGTACACACTGGTTCGTGACTCAGCGCAACTTCTACGAACCGC
AGATCATTACAACCGACAATACCTTCGTTAGCGGCAACTGTGACGTCGTTATCGGC
ATCGTGAACAACACCGTTTACGACCCGCTCCAGCCCGAGCTGGACAGTTTCAAGG
AGGAGCTGGACAAGTACTTCAAGAACCACACCAGCCCAGACGTGGACCTGGGCG
ACATCTCTGGAATCAACGCCTCCGTGGTCAACATCCAGAAGGAAATTGATCGGCTG
AACGAGGTGGCAAAGAACCTGAATGAGTCTTTGATCGATCTGCAGGAGTTGGGAA
AGTATGAGCAGTACATTAAGTGGCCATGGTATATTTGGCTGGGCTTCATTGCAGGCT
TGATCGCAATTGTGATGGTCACCATCATGCTCTGCTGCATGACTTCCTGCTGCTCTT
GTCTGAAAGGTTGTTGTAGTTGCGGTTCCTGCTGTAAGTTCGACGAGGACGATTCT
GAACCCGTGTTGAAGGGCGTTAAGCTCCACTATACCTAATAACTCGAGATGGAAATC
AAGCCAGGGCACCAACAAGTGGCCCAAGTAAGCGGCAGGCGGAAGGCCGCAGCCGC
AGCTCCCGTGGCCCAGCTGGGTCGCGTGGGCGTGGTAAATGAGTGATTCATCGTCCCA
TCTGACGAAACCCGAACTAGGCTTATGCCAGTGGT
SEQ ID NO: 6
RNA2-55/Cal09/270
Position 21: T→G mutation to delete the start codon
Position 56-1759: GOI - HA from influenza A virus/California/07/09 (H1N1) optimized for expression in humans
Position 1760-2029: final 270 nt from RNA2 wildtype 
GTAAACAACCAATAACATCAGGGTATCCAAAGCAGCACGCCGCCGAAGAGCGGCCATG
AAGGCCATCCTGGTGGTGCTGCTGTACACCTTCGCCACCGCCAACGCCGACACCC
TGTGCATCGGCTACCACGCCAACAACAGCACCGACACCGTGGACACCGTGCTGGA
GAAGAACGTGACCGTGACCCACAGCGTGAACCTGCTGGAGGACAAGCACAACGG
CAAGCTGTGCAAGCTGAGAGGCGTGGCCCCCCTGCACCTGGGCAAGTGCAACATC
GCCGGCTGGATCCTGGGCAACCCCGAGTGCGAGAGCCTGAGCACCGCCAGCAGC
TGGAGCTACATCGTGGAGACCCCCAGCAGCGACAACGGCACCTGCTACCCCGGCG
 
ACTTCATCGACTACGAGGAGCTGAGAGAGCAGCTGAGCAGCGTGAGCAGCTTCG
AGAGATTCGAGATCTTCCCCAAGACCAGCAGCTGGCCCAACCACGACAGCAACAA
GGGCGTGACCGCCGCCTGCCCCCACGCCGGCGCCAAGAGCTTCTACAAGAACCTG
ATCTGGCTGGTGAAGAAGGGCAACAGCTACCCCAAGCTGAGCAAGAGCTACATCA
ACGACAAGGGCAAGGAGGTGCTGGTGCTGTGGGGCATCCACCACCCCAGCACCA
GCGCCGACCAGCAGAGCCTGTACCAGAACGCCGACGCCTACGTGTTCGTGGGCAG
CAGCAGATACAGCAAGAAGTTCAAGCCCGAGATCGCCATCAGACCCAAGGTGAG
AGACAGAGAGGGCAGAATGAACTACTACTGGACCCTGGTGGAGCCCGGCGACAA
GATCACCTTCGAGGCCACCGGCAACCTGGTGGTGCCCAGATACGCCTTCGCCATG
GAGAGAAACGCCGGCAGCGGCATCATCATCAGCGACACCCCCGTGCACGACTGCA
ACACCACCTGCCAGACCCCCAAGGGCGCCATCAACACCAGCCTGCCCTTCCAGAA
CATCCACCCCATCACCATCGGCAAGTGCCCCAAGTACGTGAAGAGCACCAAGCTG
AGACTGGCCACCGGCCTGAGAAACATCCCCAGCATCCAGAGCAGAGGCCTGTTCG
GCGCCATCGCCGGCTTCATCGAGGGCGGCTGGACCGGCATGGTGGACGGCTGGTA
CGGCTACCACCACCAGAACGAGCAGGGCAGCGGCTACGCCGCCGACCTGAAGAG
CACCCAGAACGCCATCGACGAGATCACCAACAAGGTGAACAGCGTGATCGAGAA
GATGAACACCCAGTTCACCGCCGTGGGCAAGGAGTTCAACCACCTGGAGAAGAG
AATCGAGAACCTGAACAAGAAGGTGGACGACGGCTTCCTGGACATCTGGACCTAC
AACGCCGAGCTGCTGGTGCTGCTGGAGAACGAGAGAACCCTGGACTACCACGAC
AGCAACGTGAAGAACCTGTACGAGAAGGTGAGAAGCCAGCTGAAGAACAACGCC
AAGGAGATCGGCAACGGCTGCTTCGAGTTCTACCACAAGTGCGACAACACCTGCA
TGGAGAGCGTGAAGAACGGCACCTACGACTACCCCAAGTACAGCGAGGAGGCCA
AGCTGAACAGAGAGGAGATCGACGGCGTGAAGCTGGAGAGCACCAGAATCTACC
AGATCCTGGCCATCTACAGCACCGTGGCCAGCAGCCTGGTGCTGGTGGTGAGCCT
GGGCGCCATCAGCTTCTGGATGTGCAGCAACGGCAGCCTGCAGTGCAGAATCTGC
ATCTAATAAGTCGCTTGCAAAGACAACGCCACATTTTGGGAACGCGTCCGATCCATCCT
GAAATCTGGGCTCAACTTTGCTTCGACCATACCTGGCCCCGTAGGAGTGGCAGCGACA
GGGATCAAAGGCATCATTGAAGCCATTGGTTCCTTGTGGGTTTGATTCCACCCACAGAA
GCGTTGACGACGCAAAACGTCCTTAAAGCGTTGACGACGCAAAACGTCCCCAAGCTCG
TAGCACCGACCCTATACCCATCTCTAGGGTCTTCAACCTCTTGGT
SEQ ID NO: 7
VEEV 1^{st} RNA segment (Replicon is based on Venezuelan equine encephalitis virus strain TC-83; Gene bank MZ399799)
nsp1-4 (nt 1-7561), 11300-11446 (last 30nt from structural part plus 3'UTR), polyAtail 
ATAGGCGGCGCATGAGAGAAGCCCAGACCAATTACCTACCCAAAATGGAGAAAGT
TCACGTTGACATCGAGGAAGACAGCCCATTCCTCAGAGCTTTGCAGCGGAGCTTC
CCGCAGTTTGAGGTAGAAGCCAAGCAGGTCACTGATAATGACCATGCTAATGCCA
GAGCGTTTTCGCATCTGGCTTCAAAACTGATCGAAACGGAGGTGGACCCATCCGA
CACGATCCTTGACATTGGAAGTGCGCCCGCCCGCAGAATGTATTCTAAGCACAAGT
ATCATTGTATCTGTCCGATGAGATGTGCGGAAGATCCGGACAGATTGTATAAGTATG
CAACTAAGCTGAAGAAAAACTGTAAGGAAATAACTGATAAGGAATTGGACAAGAA
AATGAAGGAGCTGGCCGCCGTCATGAGCGACCCTGACCTGGAAACTGAGACTATG
TGCCTCCACGACGACGAGTCGTGTCGCTACGAAGGGCAAGTCGCTGTTTACCAGG
ATGTATACGCGGTTGACGGACCGACAAGTCTCTATCACCAAGCCAATAAGGGAGTT
AGAGTCGCCTACTGGATAGGCTTTGACACCACCCCTTTTATGTTTAAGAACTTGGC
TGGAGCATATCCATCATACTCTACCAACTGGGCCGACGAAACCGTGTTAACGGCTC
GTAACATAGGCCTATGCAGCTCTGACGTTATGGAGCGGTCACGTAGAGGGATGTCC
ATTCTTAGAAAGAAGTATTTGAAACCATCCAACAATGTTCTATTCTCTGTTGGCTCG
ACCATCTACCACGAGAAGAGGGACTTACTGAGGAGCTGGCACCTGCCGTCTGTAT
TTCACTTACGTGGCAAGCAAAATTACACATGTCGGTGTGAGACTATAGTTAGTTGC
GACGGGTACGTCGTTAAAAGAATAGCTATCAGTCCAGGCCTGTATGGGAAGCCTTC
AGGCTATGCTGCTACGATGCACCGCGAGGGATTCTTGTGCTGCAAAGTGACAGAC
ACATTGAACGGGGAGAGGGTCTCTTTTCCCGTGTGCACGTATGTGCCAGCTACATT
GTGTGACCAAATGACTGGCATACTGGCAACAGATGTCAGTGCGGACGACGCGCAA
AAACTGCTGGTTGGGCTCAACCAGCGTATAGTCGTCAACGGTCGCACCCAGAGAA
ACACCAATACCATGAAAAATTACCTTTTGCCCGTAGTGGCCCAGGCATTTKCTAGG
TGGGCAAAGGAATATAAGGAAGATCAAGAAGATGAAAGGCCACTAGGACTACGAG
ATAGACAGTTAGTCATGGGGTGTTGTTGGGCTTTTAGAAGGCACAAGATAACATCT
ATTTATAAGCGCCCGGATACCCAAACCATCATCAAAGTGAACAGCGATTTCCACTC
ATTCGTGCTGCCCAGGATAGGCAGTAACACATTGGAGATCGGGCTGAGAACAAGA
ATCAGGAAAATGTTAGAGGAGCACAAGGAGCCGTCACCTCTCATTACCGCCGAGG
ACGTACAAGAAGCTAAGTGCGCAGCCGATGAGGCTAAGGAGGTGCGTGAAGCCG
AGGAGTTGCGCGCAGCTCTACCACCTTTGGCAGCTGATGTTGAGGAGCCCACTCT
GGGAGCAGACGTCGACTTGATGTTACAAGAGGCTGGGGCCGGCTCAGTGGAGAC
 
ACCTCGTGGCTTGATAAAGGTTACCAGCTACGATGGCGAGGACAAGATCGGCTCTT
ACGCTGTGCTTTCTCCGCAGGCTGTACTCAAGAGTGAAAATTATCTTGCATCCAC
CCTCTCGCTGAACAAGTCATAGTGATAACACACTCTGGCCGAAAAGGCGTTATGC
CGTGGAACCATACCATGGTAAAGTAGTGGTGCCAGAGGACATGCAATACCCGTC
CAGGACTTTCAAGCTCTGAGTGAAAGTGCCACCATTGTGTACAACGAACGTGAGT
TCGTAAACAGGTVCCTGCACCATATTGCCACACATGGAGGAGCGCTGAACACTGAT
GAAGAATATTACAAAACTGTCAAGCCCAGCGAGCACGACGGCGAATACCTGTACG
ACATCGACAGGAAACAGTGCGTCAAGAAAGAACTAGTCACTGGGCTAGGGCTCA
CAGGCGAGCTGGTGGATCCTCCCTTCCATGAATTCGCCTACGAGAGTCTGAGAAC
ACGACCAGCCGCTCCTTACCAAGTACCAACCATAGGGGTGTATGGCGTGCCAGGAT
CAGGCAAGTCTGGCATCATTAAAAGCGCAGTCACCAAAAAAGATCTAGTGGTGAG
CGCCAAGAAAGAAAACTGTGCAGAAATTATAAGGGACGTCAAGAAAATGAAAGG
GCTGGACGTCAATGCCAGAACTGTGGACTCAGTGCTCTTGAATGGATGCAAACAC
CCCGTAGAGACCCTGTATATTGACGAAGCTTTTGCTTGTCATGCAGGTACTCTCAG
AGCGCTCATAGCCATTATAAGACCTAAAAAGGCAGTGCTCTGCGGGGATCCCAAAC
AGTGCGGTTTTTTAACATGATGTGCCTGAAAGTGCATTTTAACCACGAGATTTGC
ACACAAGTCTTCCACAAAAGCATCTCTCGCCGTTGCACTAAATCTGTGACTTCGGT
CGTCTCAACCTTGTTTTACGACAAAAAAATGAGAACGACGAATCCGAAAGAGACT
AAGATTGTGATTGACACTACCGGCAGTACCAAACCTAAGCAGGACGATCTCATTCT
CACTTGTTTCAGAGGGTGGGTGAAGCAGTTGCAAATAGATTACAAAGGCAACGAA
ATAATGACGGCAGCTGCCTCTCAAGGGCTGACCCGTAAAGGTGTGTATGCCGTTCG
GTACAAGGTGAATGAAAATCCTCTGTACGCACCCACCTCAGAACATGTGAACGTC
CTACTGACCCGCACGGAGGACCGCATCGTGTGGAAAACACTAGCCGGCGACCCAT
GGATAAAAACACTGACTGCCAAGTACCCTGGGAATTTCACTGCCACGATAGAGGA
GTGGCAAGCAGAGCATGATGCCATCATGAGGCACATCTTGGAGAGACCGGACCCT
ACCGACGTCTTCCAGAATAAGGCAAACGTGTGTTGGGCCAAGGCTTTAGTGCCGG
TGCTGAAGACCGCTGGCATAGACATGACCACTGAACAATGGAACACTGTGGATTAT
TTTGAAACGGACAAAGCTCACTCAGCAGAGATAGTATTGAACCAACTATGCGTGA
GGTTCTTTGGACTCGATCTGGACTCCGGTCTATTTTCTGCACCCACTGTTCCGTTAT
CCATTAGGAATAATCACTGGGATAACTCCCCGTCGCCTAACATGTACGGGCTGAATA
AAGAAGTGGTCCGTCAGCTCTCTCGCAGGTACCCACAACTGCCTCGGGCAGTTGC
CACTGGAAGAGTCTATGACATGAACACTGGTACACTGCGCAATTATGATCCGCGCA
TAAACCTAGTACCTGTAAACAGAAGACTGCCTCATGCTTTAGTCCTCCACCATAAT
GAACACCCACAGAGTGACTTTTCTTCATTCGTCAGCAAATTGAAGGGCAGAACTG
 
TCCTGGTGGTCGGGGAAAAGTTGTCCGTCCCAGGCAAAATGGTTGACTGGTTGTC
AGACCGGCCTGAGGCTACCTTCAGAGCTCGGCTGGATTTAGGCATCCCAGGTGATG
TGCCCAAATATGACATAATATTTGTTAATGTGAGGACCCCATATAAATACCATCACTA
TCAGCAGTGTGAAGACCATGCCATTAAGCTTAGCATGTTGACCAAGAAAGCTTGTC
TGCATCTGAATCCCGGCGGAACCTGTGTCAGCATAGGTTATGGTTACGCTGACAGG
GCCAGCGAAAGCATCATTGGTGCTATAGCGCGGCAGTTCAAGTTTTCCCGGGTATG
CAAACCGAAATCCTCACTTGAAGAGACGGAAGTTCTGTTTGTATTCATTGGGTACG
ATCGCAAGGCCCGTACGCACAATCCTTACAAGCTTTCATCAACCTTGACCAACATT
TATACAGGTTCCAGACTCCACGAAGCCGGATGTGCACCCTCATATCATGTGGTGCG
AGGGGATATTGCCACGGCCACCGAAGGAGTGATTATAAATGCTGCTAACAGCAAA
GGACAACCTGGCGGAGGGGTGTGCGGAGCGCTGTATAAGAAATTCCCGGAAAGCT
TCGATTTACAGCCGATCGAAGTAGGAAAAGCGCGACTGGTCAAAGGTGCAGCTAA
ACATATCATTCATGCCGTAGGACCAAACTTCAACAAAGTTTCGGAGGTTGAAGGTG
ACAAACAGTTGGCAGAGGCTTATGAGTCCATCGCTAAGATTGTCAACGATAACAAT
TACAAGTCAGTAGCGATTCCACTGTTGTCCACCGGCATCTTTTCCGGGAACAAAGA
TCGACTAACCCAATCATTGAACCATTTGCTGACAGCTTTAGACACCACTGATGCAG
ATGTAGCCATATACTGCAGGGACAAGAAATGGGAAATGACTCTCAAGGAAGCAGT
GGCTAGGAGAGAAGCAGTGGAGGAGATATGCATATCCGACGACTCTTCAGTGACA
GAACCTGATGCAGAGCTGGTGAGGGTGCATCCGAAGAGTTCTTTGGCTGGAAGGA
AGGGCTACAGCACAAGCGATGGCAAAACTTTCTCATATTTGGAAGGGACCAAGTT
TCACCAGGCGGCCAAGGATATAGCAGAAATTAATGCCATGTGGCCCGTTGCAACGG
AGGCCAATGAGCAGGTATGCATGTATATCCTCGGAGAAAGCATGAGCAGTATTAGG
TCGAAATGCCCCGTCGAAGAGTCGGAAGCCTCCACACCACCTAGCACGCTGCCTT
GCTTGTGCATCCATGCCATGACTCCAGAAAGAGTACAGCGCCTAAAAGCCTCACGT
CCAGAACAAATTACTGTGTGCTCATCCTTTCCATTGCCGAAGTATAGAATCACTGGT
GTGCAGAAGATCCAATGCTCCCAGCCTATATTGTTCTCACCGAAAGTGCCTGCGTA
TATTCATCCAAGGAAGTATCTCGTGGAAACACCACCGGTAGACGAGACTCCGGAG
CCATCGGCAGAGAACCAATCCACAGAGGGGACACCTGAACAACCACCACTTATAA
CCGAGGATGAGACCAGGACTAGAACGCCTGAGCCGATCATCATCGAAGAGGAAG
AAGAGGATAGCATAAGTTTGCTGTCAGATGGCCCGACCCACCAGGTGCTGCAAGT
CGAGGCAGACATTCACGGGCCGCCCTCTGTATCTAGCTCATCCTGGTCCATTCCTC
ATGCATCCGACTTTGATGTGGACAGTTTATCCATACTTGACACCCTGGAGGGAGCT
AGCGTGACCAGCGGGGCAACGTCAGCCGAGACTAACTCTTACTTCGCAAAGAGTA
TGGAGTTTCTGGCGCGACCGGTGCCTGCGCCTCGAACAGTATTCAGGAACCCTCC
 
ACATCCCGCYCCGCGCACAAGAACACCGTCACTTGCACCCAGCAGGGCCTGCTCG
AGAACCAGCCTAGTTTCCACCCCGCCAGGCGTGAATAGGGTGATCACTAGAGAGG
AGCTCGAGGCGCTTACCCCGTCACGCACTCCTAGCAGGTCGGTCTCGAGAACCAG
CCTGGTCTCCAACCCGCCAGGCGTAAATAGGGTGATTACAAGAGAGGAGTTTGAG
GCGTTCGTAGCACAACAACAATGACGGTTTGATGCGGGTGCATACATCTTTTCCTC
CGACACCGGTCAAGGGCATTTACAACAAAAATCAGTAAGGCAAACGGTGCTATCC
GAAGTGGTGTTGGAGAGGACCGAATTGGAGATTTCGTATGCCCCGCGCCTCGACC
AAGAAAAAGAAGAATTACTACGCAAGAAATTACAGTTAAATCCCACACCTGCTAA
CAGAAGCAGATACCAGTCCAGGAAGGTGGAGAACATGAAAGCCATAACAGCTAG
ACGTATTCTGCAAGGCCTAGGGCATTATTGAAGGCAGAAGGAAAAGTGGAGTGC
TACCGAACCCTGCATCCTGTTCCTTTGTATTCATCTAGTGTGAACCGTGCCTTTTCA
AGCCCCAAGGTCGCAGTGGAAGCCTGTAACGCCATGTTGAAAGAGAACTTTCCGA
CTGTGGCTTCTTACTGTATTATTCCAGAGTACGATGCCTATTTGGACATGGTTGACG
GAGCTTCATGCTGCTTAGACACTGCCAGTTTTGCCCTGCAAAGCTGCGCAGCTTT
CCAAAGAAACACTCCTATTTGGAACCCACAATACGATCGGCAGTGCCTTCAGCGAT
CCAGAACACGCTCCAGAACGTCCTGGCAGCTGCCACAAAAAGAAATTGCAATGTC
ACGCAAATGAGAGAATTGCCCGTATTGGATTCGGCGGCCTTTAATGTGGAATGCTT
CAAGAAATATGCGTGTAATAATGAATATTGGGAAACGTTTAAAGAAAACCCCATCA
GGCTTACTGAAGAAAACGTGGTAAATTACATTACCAAATTAAAAGGACCAAAAGC
TGCTGCTCTTTTTGCGAAGACACATAATTTGAATATGTTGCAGGACATACCAATGGA
CAGGTTTGTAATGGACTTAAAGAGAGACGTGAAAGTGACTCCAGGAACAAAACAT
ACTGAAGAACGGCCCAAGGTACAGGTGATCCAGGCTGCCGATCCGCTAGCAACAG
CGTATCTGTGCGGAATCCACCGAGAGCTGGTTAGGAGATTAAATGCGGTCCTGCTT
CCGAACATTCATACACTGTTTGATATGTCGGCTGAAGACTTTGACGCTATTATAGCC
GAGCACTTCCAGCCTGGGGATTGTGTTCTGGAAACTGACATCGCGTCGTTTGATAA
AAGTGAGGACGACGCCATGGCTCTGACCGCGTTAATGATTCTGGAAGACTTAGGT
GTGGACGCAGAGCTGTTGACGCTGATTGAGGCGGCTTTCGGCGAAATTTCATCAAT
ACATTTGCCCACTAAAACTAAATTTAAATTCGGAGCCATGATGAAATCTGGAATGTT
CCTCACACTGTTTGTGAACACAGTCATTAACATTGTAATCGCAAGCAGAGTGTTGA
GAGAACGGCTAACCGGATCACCATGTGCAGCATTCATTGGAGATGACAATATCGTG
AAAGGAGTCAAATCGGACAAATTAATGGCAGACAGGTGCGCCACCTGGTTGAATA
TGGAAGTCAAGATTATAGATGCTGTGGTGGGCGAGAAAGCGCCTTATTTCTGTGGA
GGGTTTATTTTGTGTGACTCCGTGACCGGCACAGCGTGCCGTGTGGCAGACCCCCT
AAAAAGGCTGTTTAAGCTTGGCAAACCTCTGGCAGCAGACGATGAACATGATGAT
 
GACAGGAGAAGGGCATTGCATGAAGAGTCAACACGCTGGAACCGAGTGGGTATTC
TTTCAGAGCTGTGCAAGGCAGTAGAATCAAGGTATGAAACCGTAGGAACTTCCAT
CATAGTTATGGCCATGACTACTCTAGCTAGCAGTGTTAAATCATTCAGCTACCTGAG
AGGGGCCCCTATAACTCTCTACGGCTAACCTGAATGGACTACGACATAGTCTAGTC
CGCCAAGTACGTGCTGACCAACCAGAAACATAATTGAATACAGCAGCAATTGGCA
AGCTGCTTACATAGAACTCGCGGCGATTGGCATGCCGCCTTAAAATTTTATTTTAT
TTTTCTTTTCTTTTCCGAATCGGATTTTGTTTTTAATATTTCAAAAAn
SEQ ID NO: 8
VEEV 2^{nd} RNA segment for the expression of heterologous protein (GOI, HA from influenza virus A/Cal/09/07 codon optimized)
1-44 5'UTR
45-147 CSE nsp1 (T→G in position 46 to mutate start codon)
148-1850 A/California/07/09 (H1N1) optimized
1851-1920 SGP
1921-2037 3'UTR
AAAn polyA tail 
ATAGGCGGCGCATGAGAGAAGCCCAGACCAATTACCTACCCAAAAGGAGAAAGTT
CACGTTGACATCGAGGAAGACAGCCCATTCCTCAGAGCTTTGCAGCGGAGCTTCC
CGCAGTTTGAGGTAGAAGCCAAGCAGGTCACTGATAATGAAGGCCATCCTGGTGG
TGCTGCTGTACACCTTCGCCACCGCCAACGCCGACACCCTGTGCATCGGCTACCAC
GCCAACAACAGCACCGACACCGTGGACACCGTGCTGGAGAAGAACGTGACCGTG
ACCCACAGCGTGAACCTGCTGGAGGACAAGCACAACGGCAAGCTGTGCAAGCTG
AGAGGCGTGGCCCCCCTGCACCTGGGCAAGTGCAACATCGCCGGCTGGATCCTGG
GCAACCCCGAGTGCGAGAGCCTGAGCACCGCCAGCAGCTGGAGCTACATCGTGG
AGACCCCCAGCAGCGACAACGGCACCTGCTACCCCGGCGACTTCATCGACTACGA
GGAGCTGAGAGAGCAGCTGAGCAGCGTGAGCAGCTTCGAGAGATTCGAGATCTTC
CCCAAGACCAGCAGCTGGCCCAACCACGACAGCAACAAGGGCGTGACCGCCGCC
TGCCCCCACGCCGGCGCCAAGAGCTTCTACAAGAACCTGATCTGGCTGGTGAAGA
AGGGCAACAGCTACCCCAAGCTGAGCAAGAGCTACATCAACGACAAGGGCAAGG
AGGTGCTGGTGCTGTGGGGCATCCACCACCCCAGCACCAGCGCCGACCAGCAGA
GCCTGTACCAGAACGCCGACGCCTACGTGTTCGTGGGCAGCAGCAGATACAGCAA
GAAGTTCAAGCCCGAGATCGCCATCAGACCCAAGGTGAGAGACAGAGAGGGCAG
AATGAACTACTACTGGACCCTGGTGGAGCCCGGCGACAAGATCACCTTCGAGGCC
ACCGGCAACCTGGTGGTGCCCAGATACGCCTTCGCCATGGAGAGAAACGCCGGCA
 
GCGGCATCATCATCAGCGACACCCCCGTGCACGACTGCAACACCACCTGCCAGAC
CCCCAAGGGCGCCATCAACACCAGCCTGCCCTTCCAGAACATCCACCCCATCACC
ATCGGCAAGTGCCCCAAGTACGTGAAGAGCACCAAGCTGAGACTGGCCACCGGC
CTGAGAAACATCCCCAGCATCCAGAGCAGAGGCCTGTTCGGCGCCATCGCCGGCT
TCATCGAGGGCGGCTGGACCGGCATGGTGGACGGCTGGTACGGCTACCACCACCA
GAACGAGCAGGGCAGCGGCTACGCCGCCGACCTGAAGAGCACCCAGAACGCCAT
CGACGAGATCACCAACAAGGTGAACAGCGTGATCGAGAAGATGAACACCCAGTT
CACCGCCGTGGGCAAGGAGTTCAACCACCTGGAGAAGAGAATCGAGAACCTGAA
CAAGAAGGTGGACGACGGCTTCCTGGACATCTGGACCTACAACGCCGAGCTGCTG
GTGCTGCTGGAGAACGAGAGAACCCTGGACTACCACGACAGCAACGTGAAGAAC
CTGTACGAGAAGGTGAGAAGCCAGCTGAAGAACAACGCCAAGGAGATCGGCAAC
GGCTGCTTCGAGTTCTACCACAAGTGCGACAACACCTGCATGGAGAGCGTGAAGA
ACGGCACCTACGACTACCCCAAGTACAGCGAGGAGGCCAAGCTGAACAGAGAGG
AGATCGACGGCGTGAAGCTGGAGAGCACCAGAATCTACCAGATCCTGGCCATCTA
CAGCACCGTGGCCAGCAGCCTGGTGCTGGTGGTGAGCCTGGGCGCCATCAGCTTC
TGGATGTGCAGCAACGGCAGCCTGCAGTGCAGAATCTGCATCTAATAACCTGAATG
GACTACGACATAGTCTAGTCCGCCAAGATACAGCAGCAATTGGCAAGCTGCTTACA
TAGAACTCGCGGCGATTGGCATGCCGCCTTAAAATTTTTATTTTATTTTTCTTTTCTT
TTCCGAATCGGATTTTGTTTTTAATATTTCAAAn
SEQ ID NO: 9
VEEV RNA segment encoding GOI (influenza A virus HA) inserted after the SGP and huge parts of nsp1-4 deleted
1-44 5'UTR
45-146 CSE nsp1 (mutation T→G in position 46 to destroy start codon)
147-228 last 29 nt from nsp4, SGP, 18 nt from structural region
229-1926 A/California/07/09 (H1N1) optimized
1927-1962 final 30nt from structural region
1963-2079 3'UTR
2080 - polyAtail 
ATAGGCGGCGCATGAGAGAAGCCAGACCAATTACCTACCCAAAAGGAGAAAGTT
CACGTTGACATCGAGGAAGACAGCCCATTCCTCAGAGCTTTGCAGCGGAGCTTCC
CGCAGTTTGAGGTAGAAGCCAAGCAGGTCACTGATAGAGGGGCCCCTATAACTCT
CTACGGCTAACCTGAATGGACTACGACATAGTCTAGTCCGCCAAGAGGTTCCCGTT
CCAGCCAATGAAGGCCATCCTGGTGGTGCTGCTGTACACCTTCGCCACCGCCAAC
 
GCCGACACCCTGTGCATCGGCTACCACGCCAACAACAGCACCGACACCGTGGACA
CCGTGCTGGAGAAGAACGTGACCGTGACCCACAGCGTGAACCTGCTGGAGGACA
AGCACAACGGCAAGCTGTGCAAGCTGAGAGGCGTGGCCCCCCTGCACCTGGGCA
AGTGCAACATCGCCGGCTGGATCCTGGGCAACCCCGAGTGCGAGAGCCTGAGCAC
CGCCAGCAGCTGGAGCTACATCGTGGAGACCCCCAGCAGCGACAACGGCACCTG
CTACCCCGGCGACTTCATCGACTACGAGGAGCTGAGAGAGCAGCTGAGCAGCGTG
AGCAGCTTCGAGAGATTCGAGATCTTCCCCAAGACCAGCAGCTGGCCCAACCACG
ACAGCAACAAGGGCGTGACCGCCGCCTGCCCCCACGCCGGCGCCAAGAGCTTCT
ACAAGAACCTGATCTGGCTGGTGAAGAAGGGCAACAGCTACCCCAAGCTGAGCA
AGAGCTACATCAACGACAAGGGCAAGGAGGTGCTGGTGCTGTGGGGCATCCACC
ACCCCAGCACCAGCGCCGACCAGCAGAGCCTGTACCAGAACGCCGACGCCTACGT
GTTCGTGGGCAGCAGCAGATACAGCAAGAAGTTCAAGCCCGAGATCGCCATCAGA
CCCAAGGTGAGAGACAGAGAGGGCAGAATGAACTACTACTGGACCCTGGTGGAG
CCCGGCGACAAGATCACCTTCGAGGCCACCGGCAACCTGGTGGTGCCCAGATACG
CCTTCGCCATGGAGAGAAACGCCGGCAGCGGCATCATCATCAGCGACACCCCCGT
GCACGACTGCAACACCACCTGCCAGACCCCCAAGGGCGCCATCAACACCAGCCTG
CCCTTCCAGAACATCCACCCCATCACCATCGGCAAGTGCCCCAAGTACGTGAAGA
GCACCAAGCTGAGACTGGCCACCGGCCTGAGAAACATCCCCAGCATCCAGAGCA
GAGGCCTGTTCGGCGCCATCGCCGGCTTCATCGAGGGCGGCTGGACCGGCATGGT
GGACGGCTGGTACGGCTACCACCACCAGAACGAGCAGGGCAGCGGCTACGCCGC
CGACCTGAAGAGCACCCAGAACGCCATCGACGAGATCACCAACAAGGTGAACAG
CGTGATCGAGAAGATGAACACCCAGTTCACCGCCGTGGGCAAGGAGTTCAACCAC
CTGGAGAAGAGAATCGAGAACCTGAACAAGAAGGTGGACGACGGCTTCCTGGAC
ATCTGGACCTACAACGCCGAGCTGCTGGTGCTGCTGGAGAACGAGAGAACCCTGG
ACTACCACGACAGCAACGTGAAGAACCTGTACGAGAAGGTGAGAAGCCAGCTGA
AGAACAACGCCAAGGAGATCGGCAACGGCTGCTTCGAGTTCTACCACAAGTGCG
ACAACACCTGCATGGAGAGCGTGAAGAACGGCACCTACGACTACCCCAAGTACAG
CGAGGAGGCCAAGCTGAACAGAGAGGAGATCGACGGCGTGAAGCTGGAGAGCA
CCAGAATCTACCAGATCCTGGCCATCTACAGCACCGTGGCCAGCAGCCTGGTGCTG
GTGGTGAGCCTGGGCGCCATCAGCTTCTGGATGTGCAGCAACGGCAGCCTGCAGT
GCAGAATCTGCATCTAATAATACGTGCTGACCAACCAGAAACATAATTGAATACAG
CAGCAATTGGCAAGCTGCTTACATAGAACTCGCGGCGATTGGCATGCCGCCTTAAA
ATTTTTATTTTATTTTTCTTTTCTTTTCCGAATCGGATTTTGTTTTTAATATTTCAAAA
An
SEQ ID NO: 10
nrRNA-HA1
HA from influenza A virus/California/07/09 (H1N1) optimized for expression in humans

GTAAACAACCAATAACATCAGGGTATCCAAAGCAGCACGCCGCCGAAGAGCGGCCATG
AAGGCCATCCTGGTGGTGCTGCTGTACACCTTCGCCACCGCCAACGCCGACACCC
TGTGCATCGGCTACCACGCCAACAACAGCACCGACACCGTGGACACCGTGCTGGA
GAAGAACGTGACCGTGACCCACAGCGTGAACCTGCTGGAGGACAAGCACAACGG
CAAGCTGTGCAAGCTGAGAGGCGTGGCCCCCCTGCACCTGGGCAAGTGCAACATC
GCCGGCTGGATCCTGGGCAACCCCGAGTGCGAGAGCCTGAGCACCGCCAGCAGC
TGGAGCTACATCGTGGAGACCCCCAGCAGCGACAACGGCACCTGCTACCCCGGCG
ACTTCATCGACTACGAGGAGCTGAGAGAGCAGCTGAGCAGCGTGAGCAGCTTCG
AGAGATTCGAGATCTTCCCCAAGACCAGCAGCTGGCCCAACCACGACAGCAACAA
GGGCGTGACCGCCGCCTGCCCCCACGCCGGCGCCAAGAGCTTCTACAAGAACCTG
ATCTGGCTGGTGAAGAAGGGCAACAGCTACCCCAAGCTGAGCAAGAGCTACATCA
ACGACAAGGGCAAGGAGGTGCTGGTGCTGTGGGGCATCCACCACCCCAGCACCA
GCGCCGACCAGCAGAGCCTGTACCAGAACGCCGACGCCTACGTGTTCGTGGGCAG
CAGCAGATACAGCAAGAAGTTCAAGCCCGAGATCGCCATCAGACCCAAGGTGAG
AGACAGAGAGGGCAGAATGAACTACTACTGGACCCTGGTGGAGCCCGGCGACAA
GATCACCTTCGAGGCCACCGGCAACCTGGTGGTGCCCAGATACGCCTTCGCCATG
GAGAGAAACGCCGGCAGCGGCATCATCATCAGCGACACCCCCGTGCACGACTGCA
ACACCACCTGCCAGACCCCCAAGGGCGCCATCAACACCAGCCTGCCCTTCCAGAA
CATCCACCCCATCACCATCGGCAAGTGCCCCAAGTACGTGAAGAGCACCAAGCTG
AGACTGGCCACCGGCCTGAGAAACATCCCCAGCATCCAGAGCAGAGGCCTGTTCG
GCGCCATCGCCGGCTTCATCGAGGGCGGCTGGACCGGCATGGTGGACGGCTGGTA
CGGCTACCACCACCAGAACGAGCAGGGCAGCGGCTACGCCGCCGACCTGAAGAG
CACCCAGAACGCCATCGACGAGATCACCAACAAGGTGAACAGCGTGATCGAGAA
GATGAACACCCAGTTCACCGCCGTGGGCAAGGAGTTCAACCACCTGGAGAAGAG
AATCGAGAACCTGAACAAGAAGGTGGACGACGGCTTCCTGGACATCTGGACCTAC
AACGCCGAGCTGCTGGTGCTGCTGGAGAACGAGAGAACCCTGGACTACCACGAC
AGCAACGTGAAGAACCTGTACGAGAAGGTGAGAAGCCAGCTGAAGAACAACGCC
AAGGAGATCGGCAACGGCTGCTTCGAGTTCTACCACAAGTGCGACAACACCTGCA
TGGAGAGCGTGAAGAACGGCACCTACGACTACCCCAAGTACAGCGAGGAGGCCA
AGCTGAACAGAGAGGAGATCGACGGCGTGAAGCTGGAGAGCACCAGAATCTACC
AGATCCTGGCCATCTACAGCACCGTGGCCAGCAGCCTGGTGCTGGTGGTGAGCCT

GGGCGCCATCAGCTTCTGGATGTGCAGCAACGGCAGCCTGCAGTGCAGAATCTGC
ATCTAATAAGTCGCTTGCAAAGACAACGCCACATTTTGGAACGCGTCCGATCCATCCT
GAAATCTGGGCTCAACTTTGCTTCGACCATACCTGGCCCCGTAGGAGTGGCAGCGACA
GGGATCAAAGGCATCATTGAAGCCATTGGTTCCTTGTGGGTTTGATTCCACCCACAGAA
GCGTTGACGACGCAAAACGTCCTTAAAGCGTTGACGACGCAAAACGTCCCCAAGCTCG
TAGCACCGACCCTATACCCATCTCTAGGGTCTTCAACCTCTTGGT

| Table 3 Neoantigen vaccine sequences for inclusion in a transreplicon according to the invention for vaccination of a patient with a metastatic gastrointestinal cancer: | | | |
|---|---|---|---|
| **Gene name** | **Mutation** | **Mut epitope** | **SEQ ID NO:** |
| TRAFD1 | R11L | MAEFLDDQETLLCDNCKKEIPVF | 11 |
| HNRNPU | F580I | AAQRRKMCLFAGIQRKAVVVCPKD E | 12 |
| RNF213 | P4766H | HIVEQKNGKERVHILWHFLQKEAE L | 13 |
| FMOD | S332N | LQGNRINEFSISNFCTVVDVVNFSK | 14 |
| METTL2B | R166W | LRDYGRYDMAQLWFKKGQCLSGN FY | 15 |
| PRLR | E116K | NATNQMGSSFSDKLYVDVTYIVQP D | 16 |
| SPEN | T1260K | KVDEKVLPYSNIKVREESLKFNPYD | 17 |

Plug and Play system for the generation of DNA that encodes a NoV split replicon:
1^{st} RNA segment: saRdRp which is RNA1 wt for the Nodamura virus system (SEQ ID NO: 1)
2^{nd} RNA segment: RNA polymerase promoter site like T7, SP6, T3, etc, (or similar for start of RNA transcription) followed by 21 nucleotides from the 5'end of NoV, followed by the gene(s) of interest, followed by the 3' 164 nucleotides from NoV.

### Sequences

5' sequence: T7+5'UTR ... GOI.... 3' sequence (final 164 nt)
SEQ ID NO: 18
   TAATACGACTCACTATAGTATTGAATCCAAAACTCAAA
followed by nucleic acid encoding GOI that precedes
SEQ ID NO: 19 
CTCGAGATGGAAATCAAGCCAGGGCACCAACAAGTGGCCCAAGTAAGCGGCAGG
CGGAAGGCCGCAGCCGCAGCTCCCGTGGCCCAGCTGGGTCGCGTGGGCGTGGTA
AATGAGTGATTCATCGTCCCATCTGACGAAACCCGAACTAGGCTTATGCCAGTGGT
Plug and Play system for the generation of DNA that encodes a VEEV TC-83 alphavirus split replicon:
Segment 1:
   SEQ ID NO: 7
   VEEV 1^{st} RNA segment (Replicon is based on Venezuelan equine encephalitis virus strain TC-83; Gene bank MZ399799)
   nsp1-4 (nt 1-7561), 11300-11446 (last 30nt from structural part plus 3'UTR), polyAtail
Segment 2:
   T7, 5'UTR, CSE (T→G in position 64 to mutate start codon) ... GOI... SGP, 3'UTR, polyA SEQ ID NO: 20 
TAATACGACTCACTATATATAGGCGGCGCATGAGAGAAGCCCAGACCAATTACCTACCC
AAAAGGAGAAAGTTCACGTTGACATCGAGGAAGACAGCCCATTCCTCAGAGCTTT
GCAGCGGAGCTTCCCGCAGTTTGAGGTAGAAGCCAAGCAGGTCACTGATA
   followed by nucleic acid encoding GOI that precedes
   SEQ ID NO: 21

CCTGAATGGACTACGACATAGTCTAGTCCGCCAAGATACAGCAGCAATTGGCAAGC
TGCTTACATAGAACTCGCGGCGATTGGCATGCCGCCTTAAAATTTTATTTTATTTTT
CTTTTCTTTTCCGAATCGGATTTGTTTTAATATTTCAAAₙ

## Claims

1. A split replicon system allowing gene-of-interest (GOI) multivalency comprising ribonucleic acid (RNA) derived from a virus selected from any of the group of positive strand RNA-viruses,
a. said system comprising at least two essentially purified ribonucleic acids (RNAs) derived from said virus from each of which RNA viral structural protein genes are at least functionally deleted, each provided with essentially the same 5' - and a 3' regulatory sequences derived from said virus,
b. wherein a first RNA, herein identified as self-amplifying (sa)RdRp-RNA or cisreplicon (CR), encodes a functional RNA-dependent RNA polymerase (RdRp)-gene derived from said virus and allows self-amplification *in cis* by said RdRp derived from said first RNA,
c. and wherein at least a second RNA, herein identified as GOI#1-RNA or transreplicon (TR) and essentially derivable from the first RNA wherein said RdPp-gene is at least partly replaced with a ribonucleic acid encoding a first gene-of interest (GOI#1) allows amplification of said GOI#1 *in trans* by said RdRp derived from said first RNA.

2. The system according to claim 1 comprising at least a third essentially purified RNA, herein identified as GOI#2-RNA, essentially derivable from the first RNA wherein said RdPp-gene is at least partly replaced with a ribonucleic acid encoding a second gene-of interest, allowing amplification of said GOI#2 *in trans.*

3. The system according to claim 2 comprising at least a fourth essentially purified RNA, herein identified as GOI#3-RNA, essentially derivable from the first RNA wherein said RdPp-gene is at least partly replaced with a ribonucleic acid encoding a third gene-of interest, allowing amplification of said GOI#3 *in trans.*

4. The system according to any of claims 1-3 wherein said 5'-UTR is followed by a first conserved sequence element (CSE) essentially derived from said virus.

5. The system according to any of claims 1-4 wherein a GOI is inserted upstream of a subgenomic promotor region (SGP) of a virus provided with an SGP.

6. The system according to any of claims 1-4 wherein said positive strand RNA virus is a bicistronic or bipartite virus.

7. The system of claim 6 wherein a GOI is inserted in an RpRd-encoding RNA segment of a bicistronic virus.

8. The system according to claim 7 wherein said virus is an alphavirus.

9. The system according to claim 6 wherein a GOI is inserted in an RpRd-encoding RNA segment of a bipartite virus.

10. The system according to claim 9 wherein said virus is a nodavirus.

11. A pharmaceutical formulation comprising a split replicon system according to any of claims 1 -10.

12. The formulation according to claim 11 further provided with a pharmaceutically acceptable excipient.

13. The formulation according to claim 11 or 12 further provided with a lipid nanoparticle or dendrimer formulation.

14. A vaccine comprising a formulation according to any of claims 11- 13.

15. A DNA encoding a terminal regulatory sequence of RNA of a virus covalently linked to a nucleic acid encoding a transgene wherein said DNA comprises a sequence selected from the group of SEQ ID NO: 18, SEQ ID NO: 19, SEQ ID NO: 20, and SEQ ID NO: 21
